# EUROPEAN PATENT SPECIFICATION

(11) **EP 2 253 632 B1**
(45) Date of publication and mention of the grant of the patent: **23.05.2012**
(21) Application number: 10171901.1
(22) Date of filing: 16.11.2006
(51) Int. Cl.: C07D 487/04, A61K 31/519, A61P 15/10

(54) **Pyrazolopyramidinone derivatives, their preparation and their use**
Pyrazolopyrimidinonderivate, ihre Herstellung und ihre Verwendung
Derives de la pirazolopyrimidinone, leur preparation et leur utilisation

(30) Priority: 17.11.2005 CN 200510110485
(43) Date of publication of application: 24.11.2010
(62) Divisional of application: 06817839.1
(73) Proprietor: Topharman Shanghai Co., Ltd., Shanghai 201209 (CN); Shanghai Institute of Materia Medica, Chinese Academy of Sciences, Shanghai 201203 (CN); Henan Topfond Pharmaceutical Co., Ltd., Henan 463000 (CN)
(72) Inventor: Tian, Guanghui, 201209, Pudong (CN); Lai, Shunan, 201209, Pudong (CN); Wang, Zhen, 201209, Pudong (CN); Zhu, Yi, 201209, Pudong (CN); Chen, Xinjian, 201209, Pudong (CN); Ji, Yurong, 201209, Pudong (CN); Zhang, Jinfeng, 201209, Pudong (CN); Jin, Weixi, 201209, Pudong (CN); Lv, Heping, 201209, Pudong (CN); Liu, Jinping, 201209, Pudong (CN); Wang, Wei, 201209, Pudong (CN); Ji, Ruyun, 201209, Pudong (CN); Shen, Jingshan, 201209, Pudong (CN)
(74) Representative: De Vries & Metman

(56) References cited:
- WO-A-2007/113243
- WO-A1-00/27848
- WO-A1-93/06104
- US-B1- 6 204 383
- US-B1- 6 225 315

## Description

### Technical Field

This invention relates to a series of new pyrazolopyrimidinone derivatives (1B), processes for their preparation, and pharmaceutical compositions containing them. The compounds have potent inhibitory activities against type V phosphodiesterase (PDE5), therefore they are useful for treating erectile dysfunction and other cardiovascular dysfunction.

### Background

International application WO94/28902 disclosed the use of pyrazolo[4,3-d]pyrimidine-7-one as selective cGMP PDE inhibitor for erectile dysfunction, and then WO02/27848 disclosed another series of pyrazolo[4,3-d]pyrimidine-7-one derivates, which also have potent inhibitory activity against PDE5.

The level of cGMP in the smooth muscle cell will increase once the PDE5 in smooth muscle cells are inhibited, cGMP activates protein kinase G (PKG), which subsequently phosphorylates the target protein including smooth muscle myosin, and resulting in the relaxation of smooth muscle and vasodilation. Therefore PDE5 inhibitors are useful in the treatment of a variety of cardiovascular diseases.

Sildenafil, the first launched PDE5 inhibitor, is used for male erectile dysfunction in clicnic, which also demonstrates clinical effect in female sexual dysfunction and hyperpietic. The PDE5 inhibitor under development is also used for the treatment of alimentary canal in the diabetic, insulin resistance and hyperlipemia.

Despite its effectiveness, Sildenafil has shown clinically significant adverse reactions such as headache, flushing, dyspepsia, snuffle, dimness of vision, photosensitive, and other visual disturbances, which may be linked to insufficient selectivity versus the other PDE isoforms and the dosage. Therefore, both potencies toward PDE5 and selectivities against other PDEs, especially PDE6 are the goal for the successful development of new PDE5 inhibitors

US 6,204,383 discloses that sildenafil, a known pharmaceutical chemical useful in treatment of male sexual dysfunction, is prepared by processes in which the final chemical intermediate is of significantly lower basicity than sildenafil itself, so that sildenafil can be extracted in substantially pure form from the organic reaction mixture in which it is made by adding an aqueous medium of appropriately chosen acidic pH and causing phase shift of the sildenafil to occur selectively into the aqueous phase.

WO 93/06104 discloses compounds of formula (I) 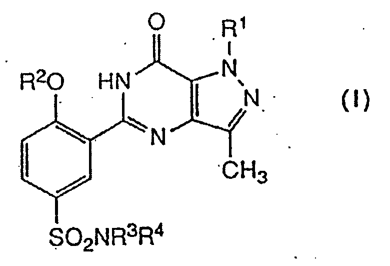 and pharmaceutically acceptable salts thereof, wherein R¹ is methyl or ethyl; R² is ethyl or n-propyl; and R³ and R⁴ are each independently H, or C₁-C₆ alkyl optionally substituted with C₅-C₇ cycloalkyl or with morpholino; said compounds being selective cGMP PDE inhibitors useful in the treatment of cardiovascular disorders such as angina, hypertension, heart failure and atherosclerosis.

US 6,225,315 relates to methods for treating nitrate-induced tolerance in a mammal by administering a nitrate-induced tolerance treating amount of a compound of formulae (I), (II), (III) (IV), (V), (VI), (VII), (VIII), (IX), (XA) or (XB) as defined herein, or the pharmaceutically acceptable salts, prodrugs, polymorphs, hydrates, solvates, active metabolites or stereoisomers thereof. The invention further relates to methods of preventing nitrate-induced tolerance in a mammal comprising administering a nitrate-induced tolerance preventing amount of a cGMP PDE inhibitor.

WO 00/27848 relates to pyrazolopyrimidinone derivatives of formula (1), their preparation method and pharmaceutical compositions containing the said derivatives. The compounds have efficacy on the treatment of impotence, one of male sexual dysfunctions with the side effects reduced.

### Summary

It is an object of the present invention to provide a series of new pyrazolopyrimidinone derivatives (1B).

It is another object of the present invention to provide the processes for the preparation of compounds of formula 1B.

It is still another object of the present invention to provide the pharmaceutical compositions containing the compounds of formula 1B.

The inventors designed and synthesized a series of novel pyrazolopyrimidinone derivatives (1B), most of which have higher inhibitory activity towards PDE5 and better selectivity against PDE6 distributing in retina than Sildenafil. Therefore, the compound provided by this invention will demonstrate better safety and efficacy, and has a good prospect in clinical application. 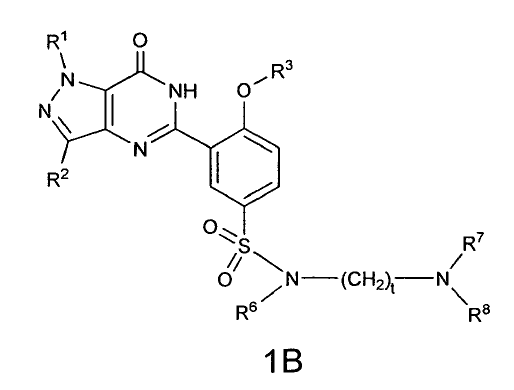 wherein:
R¹ represents H, C1-C6 alkyl, C3-C6 cycloalkyl, C1-C3 alkyl substituted by halogen or C3-C6 cycloalkyl;
R² represents C2-C6 alkyl, C3-C6 cycloalkyl, C1-C3 alkyl substituted by halogen or C3-C6 cycloalkyl;
R³ represents C1-C6 alkyl, C3-C6 cycloalkyl, C1-C3 alkyl substituted by halogen, C1-C3 alkoxyl C3-C6 cycloalkyl;
t=1-5 ;
R⁶ represents C1-C6 alkyl, C3-C6 cycloalkyl, C1-C6 haloalkyl, C1-C3 alkoxyl, phenyl, pyridyl, furanyl, pyridazinyl, pyrazinyl, imidazolyl, C₁-C₃ substituted with hydroxyl, C₁-C₃ alkoxyl, acetoxyl, phenyl, pyridyl, furanyl, pyridazinyl, pyrimidinyl, pyrazinyl, imidazolyl; the above phenyl, pyridyl, furanyl, pyridazinyl, pyrimidinyl, pyrazinyl, imidazolyl optionally substituted with one or more substituents selected from halogen, C1-C3 alkyl, C1-C3 alkoxyl;
R⁷ and R⁸ independently represent H, C₁-C₆ alkyl, C3-C6 cycloalkyl, C₁-C₃ haloalkyl, C₁-C₆ alkoxyl, C1-C3 alkyl substituted with hydroxyl, acetoxyl, C₁-C₃ alkoxyl, or R⁷ and R⁸ together with the nitrogen to which they are attached form a four-membered to eight-membered heterocyclic ring, including morpholine, piperidine, pyrrole, piperazine; the above heterocyclic ring optionally substituted with one or more substituents selected from halogen, C1-C3 alkyl, C3-C6 cycloalkyl, C₁-C₃ haloalkyl, C1-C3 alkoxyl.

As previously defined, no particular explanation, alkyl with three or more carbon wherein said alkyl may be straight or branched chain. Halogen represents fluorine, chlorine, bromine or iodine.

The compound of formula 1B may have one or more chiral center, therefore, the compound may exist as a stereomer, that is to say, enantiomer, diastereomer or their mixture. The invention includes within its scope all the possible isomers, stereomers and their mixture of formula 1B.

The compounds of formula 1B may have stereomers and this invention includes all the possible isomers, stereomers and their mixtures thereof.

The invention includes the pharmaceutically acceptable salts of formula 1B, preferred salts are hydrochloride and methanesulfonate.

The invention still includes the pharmaceutically acceptable solvates of formula 1B (e.g. hydrates).

The invention also includes the pharmaceutically oxide of formula 1B.

Preferred compounds of 1B include those wherein:
R¹ represents C₁-C₄ alkyl or C₃-C₆ cycloalkyl;
R² represents C₂-C₄ alkyl or C3-C6 cycloalkyl;
R³ represents C₁-C₃ alkyl, C₁-C₃ alkyl substituted with C₁-C₃ alkoxyl;
t=2-3 ;
R⁶ represents C₁-C₃ alkyl, phenyl, pyridyl, benzyl or C₁-C₃ substituted with hydroxyl, C₁-C₃ alkoxyl, acetoxyl, phenyl, pyridyl;
R⁷ and R⁸ together with the nitrogen to which they are attached form a morpholine, piperidine or pyrrole heterocyclic ring;

Particularly preferred compounds of 1 B include those wherein:
R¹ represents methyl or ethyl;
R² represents ethyl and n-propyl;
R³ represents ethyl, n-propyl or methyloxyethyl;
t=2-3;
R⁶ represents methyl, ethyl, benzyl, pyridylmethyl, or C₁-C₃ substituted with hydroxyl, C₁-C₃ alkoxyl, acetoxyl;

The preferable compounds of the present invention are:
1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 13)
1-methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 14)
1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one (compound of example 16)
1-ethyl-5-{2-ethoxy-5-{[1-ethyl-1-[2-(1-ethyl-1-(2-acetoxyethyl))amino]ethyl]amidosulfonyl}phenyl-3-n-propyl-1,6-dihydro-7H-pyrazolo[4,3-d]pyrimidin-7-one (compound of example 19)
1-methyl-5-{2-ethoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 21)
1-methyl-5-{2-propoxy-5-{[1-methyl-1-[2-(1-methyl-1-(2-hydroxyethyl))amino]ethyl]amidosulfonyl}phenyl-3-n-propyl-1,6-dihydro-7H-pyrazolo[4,3-d]pyrimidin-7-one (compound of example 23)
1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 24)
1-methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 25)
1-methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 26)
1-methyl-5-{2-ethoxy-5-[[1-benzyl-l-(2-morpholin-l-y1)ethyl]amidosulfonyl]phenyl}-3-ethyl-1,6-dihydro-7H-pyrazolo[4,3-d]pyrimidin-7-one (compound of example 27)
1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 28)
1 -methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one (compound of example 29)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 35)
1-methyl-5-(2-ethoxy-5-[[1-inethyl-1-(2-morpholin-1-yl)ethyl]ainidosulfonyl]phenyl)-3-ethyl-1,6-dihydro-7H-pyrazolo[4,3-d]pyrimidin-7-one(compoundofexample 36)
1-methyl-5-{2-propoxy-5-[[1-ethyl-1-(2-morpholin-1-yl)ethyl]amidosulfonyl]phenyl-3-propyl-1,6-dihydro-7H-pyrazolo[4,3-d]pyrimidin-7-one (compound of example 41)
1-methyl-5-{2-ethoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one (compound of example 43)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 53)
1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one (compound of example 54)
1-methyl-5-{2-propoxy-5-[[1-(4-fluorobenzyl)-1-(2-morpholin-1-yl)ethyl]amidosulfonyl]phenyl -3-n-propyl-1,6-dihydro-7H-pyrazolo[4,3-d]pyriinidin-7-one (compound of example 56)
1-methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 60)
1-methyl-5-{2-ethoxy-5-[[1-(3-pyridylmethyl)-1-(2-morpholin-1-yl)ethyl]amidosulfonyl]phenyl)-3-n-propyl-1,6-dihydro-7H-pyrazolo[4,3-d]pyrimidin-7-one (compound of example 61)
1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 62)
1-methyl-5-{2-propoxy-5-[[1-(4-pyridylinethyl)-1-(2-morpholin-1-yl)ethyl]amidosulfonyl]phenyl}-3-n-propyl-1,6-dihydro-7H-pyrazolo[4,3-d]pyrimidin-7-one (compound of example 63)
1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 64)
1-inethyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one (compound of example 72)
1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 78)
1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one (compound of example 79)
1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 80)
1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 81)
1-methyl-5-{2-ethoxy-5-[[1-benzyl-1-(2-piperidin-1-yl)ethyl]amidosulfonyl]phenyl-3-n-propyl-1,6-dihydro-7H-pyrazolo[4,3-d]pyrimidin-7-one (compound of example 82)
1-methyl-5-{2-propoxy-5-[[1-benzyl-1-(2-piperidin-1-yl)ethyl] amidosulfonyl]phenyl}-3-n-propyl-1,6-dihydro-7H-pyrazolo[4,3-d]pyrimidin-7-one (compound of example 83)
1-methyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 84)
1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one (compound of example 85)
1-methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 87)
1-methyl-5-{2-ethoxy-5-[[1-(2-pyridylmethyl)-1-(2-piperidin-1-yl)ethyl]amidosulfonyl]phenyl-3-n-propyl-1,6-dihydro-7H-pyrazolo[4,3-d]pyrimidin-7-one (compound of example 88)
1-methyl-5-{2-propoxy-5-[[1-(3-pyridylinethyl)-I-(2-pyrrolidin-I-yl)ethyl]amidosulfonyl]phenyl}-3-n-propyl-l,6-dihydro-7H-pyrazolo[4,3-d]pyrimidin-7-one (compound of example 89)
1-methyl-5-{2-ethoxy-5-[[1-(3-pyridylmethyl)-1-(2-pyn-olidin-1-yl)ethyl]amidosulfonyl]phenyl}-3-n-propyl-1,6-dihydro-7H-pyrazolo[4,3-d]pyrimidin-7-one (compound of example 90)
1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 95)
1-methyl-5-{2-propoxy-5-[[1-(2-acetoxyethyl)-1-(2-morpholin-1-yl)ethyl]amidosulfonyl]phenyl-3-n-propyl-1,6-dihydro-7H-pyrazolo[4,3-d]pyriinidin-7-one (compound of example 96)
1-methyl-5-{2-propoxy-5-[[1-(2-methoxyethyl)-1-(2-morpholin-1-yl)ethyl]ainidosulfonyl]phenyl}-3-n-propyl-1,6-dihydro-7H-pyrazolo[4,3-d]pyrin>idin-7-one (compound of example 98)
1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 100)
1-methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 104)
1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 105)
1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one (compound of example 110)
1-methyl-5-{2-propoxy-5-[[1-(2-ethoxyethyl)-1-(2-morpholin-1-yl)ethyl]ainidosulfonyl]phenyl}-3-n-propyl-1,6-dihydro-7H-pyrazolo[4,3-d]pyriinidin-7-one (compound of example 111)
1-methyl-5-(2-propoxy-5-[[1-ethyl-1-(3-morpholin-1-yl)propyl]amidosulfonyl]phenyl)-3-n-propyl-1,6-dihydro-7H-pyrazolo[4,3-d]pyrimidin-7-one (compound of example 112)
1-methyl-5- {2-propoxy-5-[[1-benzyl-1-(3-moipholin-1-yl)propyl]amidosulfonyl]phenyl}-3-n-propyl-1,6-dihydro-7H-pyrazolo[4,3-d]pyrimidin-7-one (compound of example 113)
1-methyl-5-{2-propoxy-5-[[1-benzyl-1-(4-morpholin-1-yl)butyl]amidosulfonyl]phenyl)-3-n-propyl-1,6-dihydro-7H-pyrazolo[4,3-d]pyrimidin-7-one (compound of example 117)

The present invention also provides the processes for the preparation of compounds of formula 1B.

Compounds of formula 1B may be prepared from 2B. The scheme is as follows: 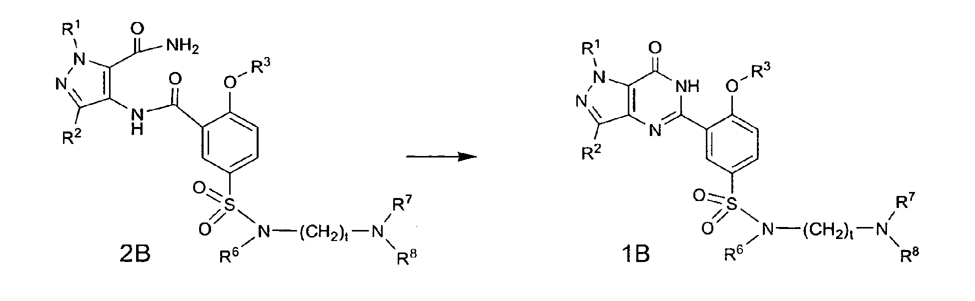 wherein R¹, R², R³, R⁶, R⁷, R⁸ and t are as previously defined for 1B.

This step was achieved by the existing cyclization method for pyrimidinone compounds. The reaction is usually carried out in the presence of a suitable base and a suitable solvent at temperatures at a range from 50 to 200°C. Preferred bases include metal alkoxides (e.g. potassium tertbutoxide, sodium ethoxide), alkaline earth metal or hydrides of alkali metal, amine (e.g. triethylamine), metal salts of ammonia, hydroxides (e.g. sodium hydroxide), carbonates and bicarbonates. Preferred solvents include alcohols (e.g. t-butanol, methanol, ethanol, isopropanol, glycol, 2-methoxyethanol), aromatic hydrocarbons (e.g. benzene, toluene, chlorobenzene), pyridine, halogenated hydrocarbon, acetonitrile, tetrahydrofuran, dioxane, dimethylsulfoxide, N,N-dimethylformamide, N-methylpyrrolidin-2-one.

The compounds of formula (2B) are usually prepared by reacting the compounds of formula (3B) with the compounds of formula 4 respectively. The scheme is as follows: 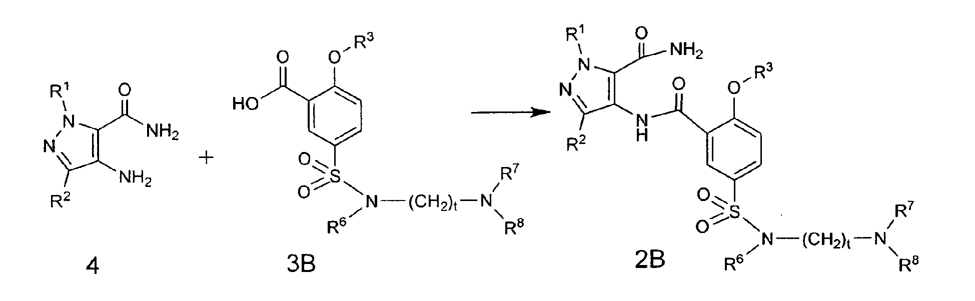 wherein R¹, R², R³, R⁶, R⁷, R⁸ and t are as previously defined for formulae 1B.

Method 1, the carboxyl group in the compounds of formula (3B) was transformed into acyl chloride or mixed anhydride by using thionyl chloride, oxalyl chloride or ethyl chlorformate, then the mixture was reacted with the compounds of formula 4 to get the corresponding acid amide (2B). The acidylation reaction is usually carried out in the presence of a suitable deacidification reagent and a common solvent. Preferred deacidification reagents include organic bases (preferred triethylamine, N,N-diisopropylethylamine, pyridine) and inorganic bases (preferred hydroxides, carbonates). Preferred solvents include diolefines (preferred petroleum, n-hexane, cyclohexane), halohydrocarbon (preferred dichlormethane, chloroform), ethers (preferred tetrahydrofuran, dioxane, ether), aromatic solvents (preferred toluene) and alcohols (preferred t-butanol, isopropanol).

Method 2, carboxylic acid was reacted with amine derivates directly to get the formula (2B). The reaction is usually carried out in the anhydrous solvent with the presence of activating agent or dehydrating agent. Preferred activating agents or dehydrating agents include DCC, EDCI, EEDQ, CDI, HOBt. Preferred solvents include halogenated hydrocarbons (e.g. dichlormethane, dichlormethane), ethers (e.g. tetrahydrofuran, dioxane, ether), aromatic hydrocarbons (e.g. benzene, toluene), polarity aprotic solvent (dimethylsulfoxide, N,N-dimethylformamide), or their mixture.

The compounds of formula 3B and 4 can be prepared according to literatures or supplied commercially.

In addition, the present invention provides the pharmaceutical compositions containing the compounds of formula 1B.

The above mentioned compositions contain one or more compounds of formula 1B (or their pharmaceutically acceptable salts, or their pharmaceutically acceptable solvates) and at least one kind of pharmaceutical excipient. The pharmaceutical excipient, which is used according the administration route and their functional properties, are normally fillers, diluents, adhesives, moistening agent, disintegrants, emulsifier, suspending agent, etc.

The compositions according to the invention can be administrated by any suitable route, for example by oral, parenteral (including intravenous, intramuscular, subcutaneous, and intracoronary), sublingual, buccal, rectal, transurethral, vaginal, nasal, inhalation or topical administration. Oral administration is the preferred route.

The compounds of formula 1B should preferably be presented in the above mentioned pharmaceutical compositions in a concentration of about 0.1 to 99.9%, preferably 1 to 99% by weight of the total mixture.

The present invention also provides processes for the preparation of the pharmaceutical compositions containing the compounds of formula 1B. The compounds of formula 1B can be mixed with pharmaceutical excipient or excipients and made into dosage forms according the administration route in the conventional method. The dosage forms include tablets, capsules, granules, pills, solutions, solutions, emulsion, emulsions, membranes, creams, aerosols, injection and suppositories etc. Tablets and capsules are preferred.

Tablets and capsules can contain one or more compounds of formula of 1 B in addition to one or more conventional excipients, such as (a) fillers, for example starches, sucrose, lactose, glucose, microcrystalline cellulose, and mannitol, (b) binders, for example carboxymethylcellulose, gelatine, alginates and polyvinylpyn-olidone, (c) humectants, for example glycerol, (d) disintegrating agents, for example agar-agar, ethyl cellulose, sodium starch glycolate and calcium carbonate (e) lubricants, for example magnesiumstearate, talc, and polyethylene glycols.

The dosages of the compounds of the invention are generally 1 to 500mg per day, preferrely 10 to 100mg, taken once or several times. However, it may be necessary to properly deviate from the dosages mentioned. The optimal dosages, which may be determined by specialist with their professional knowledge, depend on the severity of the disease, the individual response towards the medicament, the characteristics of the formulation, and the administration routes.

Moreover, the invention provides the compounds of formula 1B, or the pharmaceutically acceptable salt thereof, or the pharmaceutically acceptable solvate of either entity, or the pharmaceutical composition containing any of the foregoing, for use as a human medicament.

The invention further provides the use of the compounds of formula 1B, or the pharmaceutically acceptable salt thereof, or the pharmaceutically acceptable solvate of either entity, for the manufacture of a human medicament for the curative or prophylactic treatment of a medical condition for which a cGMP PDE5 inhibitor is indicated.

Still further, the invention provides the use of the compounds of formula 1B, or the pharmaceutically acceptable salt thereof, or the pharmaceutically acceptable solvate containing either entity, for the manufacture of a human medicament for the curative or prophylactic treatment of male erectile dysfunction, benign prostatic hyperplasia (BPH), female sexual dysfunction, premature labour, dysmenorrhoea, bladder outlet obstruction, incontinence, stable, unstable and variant (Prinzmetal) angina, hypertension, pulmonary hypertension, congestive heart failure, kidney failure, atherosclerosis, stroke, peripheral vascular disease, conditions of reduced blood vessel patency, inflammatory disease, bronchitis, chronic asthma, allergic asthma, allergic rhinitis, glaucoma or diseases characterized by disorders of gut motility (e.g. irritable bowel syndrome, IBS).

### Detailed Description of Embodiments

### Examples

The following examples serve to explain the compounds in this invention and the methods for the intermediates.

¹H NMR spectra were determined on a Mercury 400 NMR spectrometer or Mercury 400 NMR spectrometer (Varian Company). The conventional abbreviation was as follows: s, singlet; d, doublet; t, triplet; q, quartet; m, multiplet; br, broad peak.

### Example 1: 1-methyl-5-{2-propyloxy-5-[bis(2-acetoxyethyl)amidosulfonyl]phenyl}-3-n-propyl-1,6-dihydro-7H-pyrazolo[4,3-d]pyrimidin-7-one

### Step 1: preparation of 4-{2-propoxy-5-[bis(2-acetoxyethyl)amidosulfonyl]benzoylamino}-1-methyl-3-n-propylpyrazolo-5- carboxamine

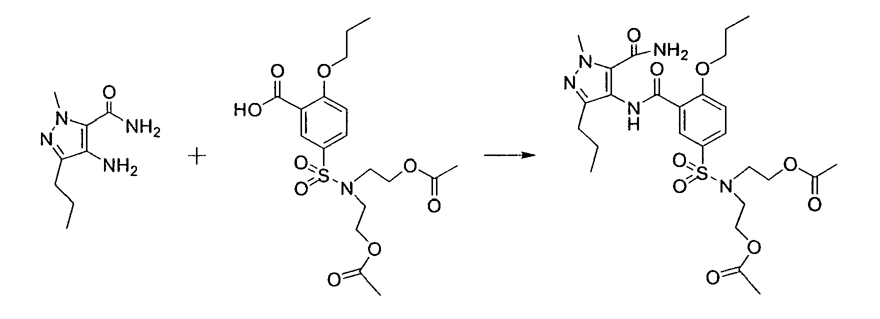

2-propoxy-5-[bis(2-acetoxyethyl)amidosulfonyl]benzoic acid (0.43g, 1mmol) was dissolved in CH₂Cl₂ (20mL), Carbonyldiimidazole (CDI, 3mmol) was added to the solution and stirred at room temperature for 0.5 hours, then 4-amino-3-propylpyrazolo-5-carboxamine (0.18g, 1 mmol) was added and stirred another 1-6h, the stopping point was detected by TLC. When the reaction was finished, the reaction mixture was washed with the ammonium chloride solution and brine, the CH₂Cl₂ layer was dried over anhydrous magnesium sulfate and the solvent was concentrated to dryness under reduced pressure, the resulting residue was recrystallized from alcohol to get white powder (0.51 g), yield 86%.

### Step 2: preparation of 1-methyl-5-{2-propoxy-5-[bis(2-acetoxyethyl)amidosulfonyl]phenyl}-3-n-propyl-1,6-dihydro-7H-pyrazolo[4,3-d]pyrimidin-7-one

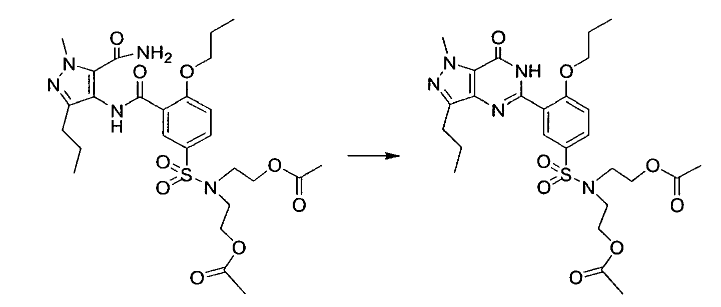

Potassium tert-butoxide (0.06g, 0.55mmol) and the product of step 1 (0.3g, 0.5mmol) were added to tert-butyl alcohol (15ml) successively, and the mixture was heated to reflux for 30 minutes to produce a clear solution. The solution was refluxed for another 10 hours, then cooled to room temperature and water (20ml) was added. The resulted solution was adjusted to neutral by adding 4% acetic acid. cooled to 5-10°C and a white solid was precipitated. The white solid was collected by filtration, washed with cold water (3×10ml) and dried. The solid was recrystallized from MeOH/EtOAc to afford the title compound (0.22g), yield 76%. ¹H NMR (CDCl₃) δ: 10.83 (1H, s), 8.87 (1H, d), 7.90 (1H, dd), 7.14 (1H, d), 4.27 (3H, s), 4.25 (6H, m), 3.49 (4H, t), 2.93 (2H, t), 2.04 (2H, m), 2.03 (6H, s), 1.86 (2H, m), 1.17 (3H, t), 1.02 (3H, t).

### Example 2: 1-methyl-5-{2-propyloxy-5-[bis(2-hydroxyethyl)amidosulfbnyl]phenyl}-3-n-propyl-1,6-dihydro-7H-pyrazolo[4,3-d]pyrimidin-7-one

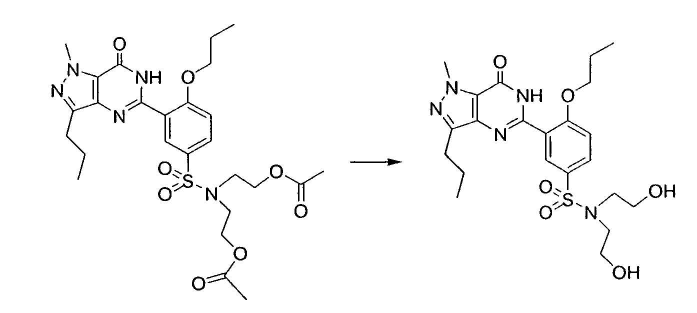

To the solution alcohol (5ml),water (10ml) and potassium carbonate (0.1 g, 0.7mmol), the compound of example 1 (0.12g, 0.2mmol) was added and the solution was heated to reflux, the stopping point was detected by TLC. When the reaction was finished, the PH value of the solution was adjusted to neutral by dilute hydrochloric acid and a white solid was precipitated, the solid was collected by filtration, washed with water and dried to get crude product. The crude product was recrystallized from dichloromethane and n-hexane to afford the title compound (0.06g), yield 61 %. H NMR (CDCl₃) δ: 10.82 (1H, s), 8.84 (1H, d), 7.91 (1H, dd), 7.14 (1 H, d), 4.26 (3H, s), 4.25 (2H, t), 3.88 (4H, t), 3.49 (2H, s), 3.38 (4H, t), 2.92 (2H, t), 2.02 (2H, m), 1.84 (2H, m), 1.17 (3H, t), 1.02 (3H, t).

### Examples 3-120:

The examples 3-120 were prepared from different substitute start materials following the procedure of example I and example 2 (unless otherwise noted, NMR spectra were determined in CDCl₃ solution)

| Example | Structure | Name and ¹H-NMR (δ) |
|---|---|---|
| 11 | 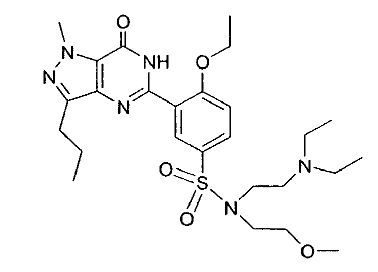 | 1-methyl-5-{2-ethoxy-5-[[1-(2-diethylaminoethyl)-1-(2-methoxyethyl)]amidosulfonyl]phenyl}-3-n-propyl-1,6-dihydro-7H-pyrazolo[4,3-d]pyiiinidin-7-one 7.97(1H, d), 7.89(1H, dd), 7.32(1H, d), 4.19(m, 2H), 4.11 (3H, s), 3.30(2H, t), 3.20(3H, s), 3.14(2H, t), 2.76(2H, t), 2.50(2H, t), 2.42(4H, m), 1.34(3H, t), 1.26(2H, m), 0.80(9H, m) (DMSO). |
| 12 | 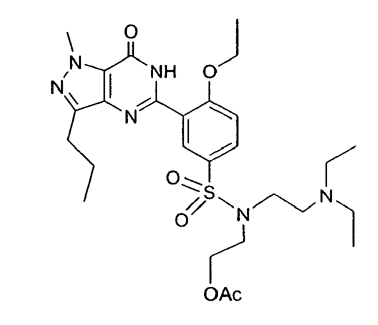 | 1-methyl-5-{2-ethoxy-5-[1-(2-diethylaminoethyl)-1-(2-acetoxyethyl)]amidosulfonyl]phenyl}-3-n-propyl-1,6-dihydro-7H-pyrazolo[4,3-d]pyrimidin-7-one 10.80(1H, s), 8.88(1H, d), 7.91(1H, dd), 7.12(1H, d), 4.39(2H, q), 4.26(3H, s), 4.23(2H, t), 3.48(2H, t), 3.30(2H, t), 2.92(2H, t), 2.70(2H, m), 2.58(4H, q), 2.04(3H, s), 1.83(2H, m), 1.63(3H, t), 1.01(9H, m) |
| 13 | 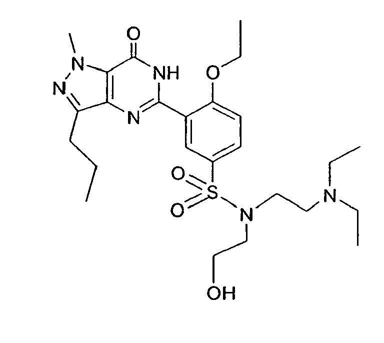 | 1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one 10.80(1H, s), 8.87 ( 1H , d ), 7.89 (1H, dd), 7.14 (1H, d), 4.37 (2H, m), 4.27 (3H, s), 3.81 (2H, t), 3.25 (4H, m), 2.93 (2H, t), 2.86 (2H, t), 2.61 (4H, t), 1.86 (2H, m), 1.64 (3H, t), 1.11 (6H, t), 1.03 (3H,t). |
| 14 | 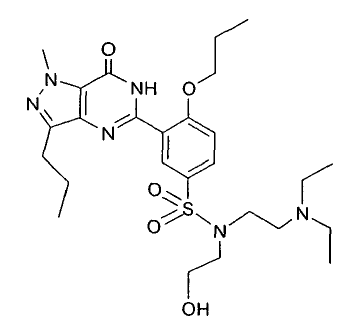 | 1-methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one 10.86(1H, s), 8.87(1H, d), 7.88(1H, dd), 7.15(1H, d), 4.29(2H, q), 4.27(3H, s), 3.67(2H, t), 3.34(2H, t), 3.24(2H, q), 2.93(2H, t), 2.61(2H, t), 2.56(2H, t), 2.53(2H, t), 2.03(2H, m), 1.85(2H, m), 1.18(3H, t), 1.11 (3H, t), 1.01(6H, t). |
| 15 | 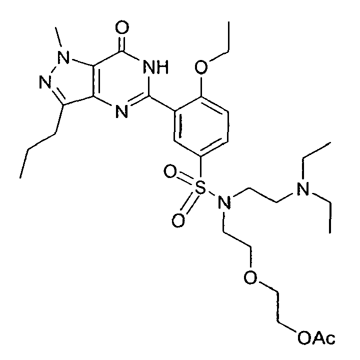 | 1-methyl-5-{2-ethoxy-5-[1-(2-diethylaminoethyl)-1-(2-acetoxyethoxyethyl)amidosulfonyl]phenyl}-3-n-propyl-1,6-dihydro-7H-pyrazolo[4,3-d]pyrimidin-7-one 10.84(1H, s), 8.88(1H, d), 7.92(1H, dd), 7.13(1H, d), 4.37(2H, q), 4.27(3H, s), 4.14(2H, t), 3.67(2H, t), 3.62(2H, q), 3.43(2H, t), 3.33(2H, t), 2.92(2H, t), 2.72(2H, m), 2.57(4H, q), 2.05(3H, s), 1.85(3H, m), 1.66(3H, t), 1.00(9H, m) |
| 16 | 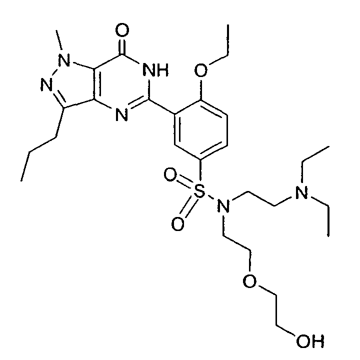 | 1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one 12.17 (1H, s), 7.97(1H , d), 7.92 (1H, dd), 7.33 (1H, d), 4.20 (2H,m), 4.16 (3H, s), 3.53 (2H, t), 3.44 (2H, t), 3.39 (2H, t), 3.30 (2H, t), 3.17 (2H, t), 2.77 (2H, t), 2.55 (2H, t), 2.44 (4H, m), 1.74 (2H, m), 1.33 (3H, t), 0.92 (9H, m)(DMSO) |
| 17 | 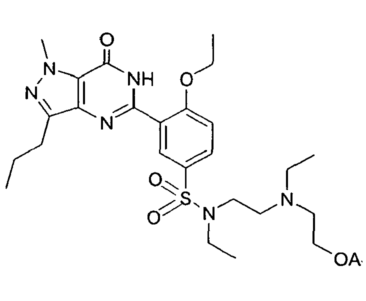 | 1-methyl-5-{2-ethoxy-5-{[1-ethyl,-1-[2-[1-ethyl-1-(2-acetoxyethyl)]amino]ethyl]amidosulfonyl]}phenyl}-3-n-propyl-1,6-dihydro-7H-pyrazolo[4,3-d]pyrimidin-7-one 10.84(1H, s), 8.88(1H, d), 7.91(1H, dd), 7.12(1H, d), 4.36(2H, q), 4.33(3H, S), 4.07(2H, t), 3.32(2H, q), 3.20(2H, t), 2.92(2H, t), 2.73(4H, m), 2.58(2H, q), 2.02(3H, s), 1.85(2H, m), 1.64(3H, t), 1.21 (3H, t), 1.01(6H, m) |
| 18 | 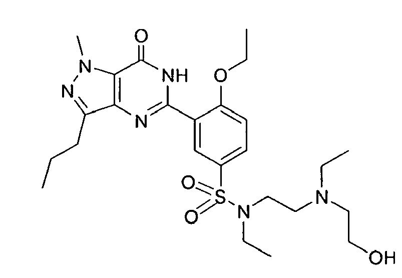 | 1-methyl-5-{2-ethoxy-5-{[1-ethyl-1-[2-[1-ethyl-1-(2-hydroxyethyl)]amino]ethyl]amidosulfonyl]}phenyl}-3-n-propyl-1,6-dihydro-7H-pyrazolo[4,3-d]pyrimidin-7-one 10.85 (1H, s), 8.87 ( 1H , d), 7.91 (1H, dd), 7.13 (1H, d), 4.37 (2H, m), 4.27 (3H, s), 3.55 (2H, t), 3.31 (2H, q), 3.24 (2H, q), 2.92 (2H, t), 2.74 (2H, t), 2.64 (2H, t), 2.62 (2H, t), 1.85 (2H, m), 1.64 (3H, t), 1.17 (3H, t), 1.03 (6H, m) |
| 19 | 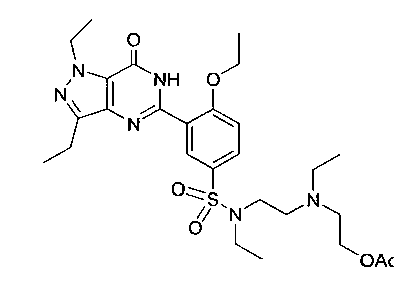 | 1-ethyl-5-{2-ethoxy-5-{[1-ethyl-1-[2-[1-ethyl-1-(2-acetoxyethyl)]amino]ethyl]amidosulfonyl}phenyl}-3-n-propyl-1,6-dihydro-7H-pyrazolo[4,3-d]pyrimidin-7-one 10.88(1H, s), 8.89(1H, d), 7.91(1H, dd), 7.13(1H, d), 4.64(2H, q), 4.37(2H, q), 4.09(2H, t), 3.32(2H, q), 3.26(2H, t), 2.98(2H, q), 2.75(4H, q), 2.62(2H, q), 2.05(3H, s), 1.65(3H, t), 1.52(3H, t), 1.41 (3H, t), 1.18(3H, t), 1.03(3H, t) |
| 20 | 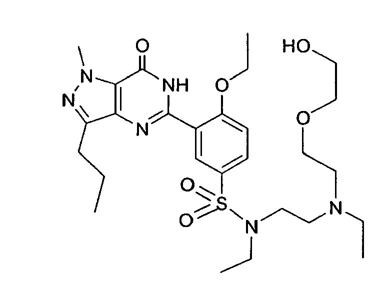 | 1-methyl-5-{2-ethoxy-5-{[1-ethyl-1-[2-[1-ethyl-1-(2-hydroxyethoxyethyl)]amino]ethyl]amidosulfonyl}phenyl}-3-n-propyl-1,6-dihydro-7H-pyrazolo[4,3-d]pyrimidin-7-one 10.84 (1 H, s), 8.85 ( 1H, d ), 7.91 (1H, dd), 7.12 (1H, d), 4.36 (2H, q), 4.27 (3H, s), 3.66 (2H, m), 3.57 (4H, m), 3.31 (2H, m), 3.24 (2H, t), 2.92 (2H, m), 2.77 (2H, t), 2.68 (2H, t), 2.60 (2H, m), 1.85 (2H, m), 1.63 (3H, t), 1.03 (3H, t). |
| 21 | 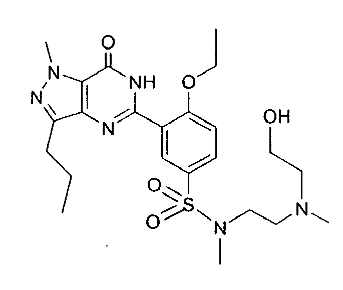 | 1-methyl-5-{2-ethoxy-5-{1-methyl-l-[2-[1-methyl-1-(2-hydroxyethyl)]amino]ethyl]amidosulfonyl}phenyl}-3-n-propyl-1,6-dihydro-7H-pyrazolo[4,3-d)pyrimidin-7-one 10.83 (1H, s), 8.84 ( 1H, d ), 7.89 (1H, dd), 7.14 (1H, d), 4.37 (2H, m), 4.27 (3H, s), 3.56 (4H, m), 2.91 (2H, t), 2.83 (3H, s), 2.66 (2H, t), 2.58 (2H, t), 2.33 (3H, s), 1.84 (2H, m), 1.64 (3H, t), 1.03(3H, t). |
| 22 | 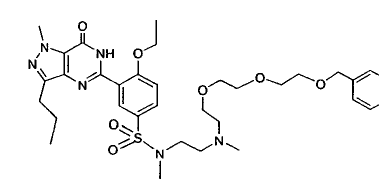 | 1-methyl-5-{2-ethoxy-5-{[1-methyl-1-[2-[1-methyl-1-(2-benzyloxyethoxyethoxyethyl)]amino]ethyl]amidosulfonyl}phenyl}-3-n-propyl-1,6-dihydro-7H-pyrazolo[4,3-d]pyrimidin-7-one 8.81 ( 1H, d ), 7.86 (1H, dd), 7.30 (5H, m), 7.12 (1H, d), 4.53 (2H, s), 4.33 (2H, m), 4.27 (3H, s), 3.62 (8H, m), 3.54 (2H, t), 3.18(2H, t), 2.92 (2H, t), 2.83 (3H, s), 2.62 (4H, m), 2.28 (3H, s), 1.85 (2H, m), 1.61 (3H, t), 1.01(3H, t). |
| 23 | 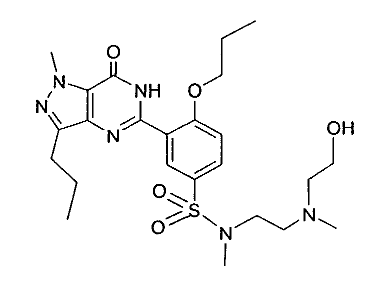 | 1-methyl-5-{2-propoxy-5-{[1-methyl-1-[2-[1-methyl-1-(2-hydroxyethyl)]amino]ethyl]amidosulfonyl}phenyl}-3-n-propyl-1,6-dihydro-7H-pyrazolo[4,3-d]pyrimidin-7-one 10.87(1H, s), 8.85(1H, d), 7.89(1H, dd), 7.16(1H, d), 4.28(3H, s), 4.27(2H, q), 3.58(2H, t), 3.24(2H, t), 2.93(2H, t), 2.83(3H, s), 2.67(2H, t), 2.59(2H, t), 2.34(3H, s), 2.04(2H, m), 1.86(2H, m), 1.19(3H, t), 1.02(3H, t) |
| 24 | 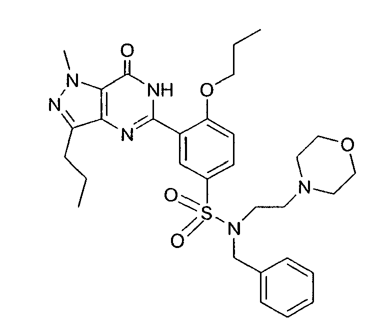 | 1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one 7.98 (2H, m), 7.35 (6H, m), 4.37 (2H, s), 4.16 (3H, s), 4.11 (2H, t), 3.86 (2H, t), 3.68 (2H, t), 3.57 (2H, t), 3.25 (2H, t), 3.10 (2H, t), 2.95 (2H, t), 2.77 (2H, t), 1.68-1.76 (4H, m), 0.89-0.96 (6H, 2xt). (DMSO) |
| 25 | 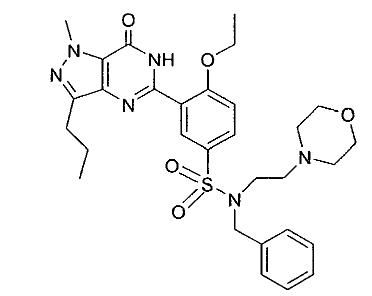 | 1-methyl-5-{2-ethoxy-5-[[1-benzyl-1-(2-morpholin-1-yl)ethyl]amidosulfonyl]phenyl}-3-n-propyl-1,6-dihydro-7H-pyrazolo [4,3-d]pyrimidin-7-one 8.01 (1H, dd), 7.96 (1H, d), 7.34 (6H, m), 4.38 (2H, s), 4.12 (3H, s), 3.88 (2H, t), 3.67 (2H, t), 3.55 (2H, t), 3.26 (2H, t), 3.11 (2H, t), 2.97 (2H, t), 2.78 (2H, t), 1.71 (2H, m), 0.92 (3H, t). (DMSO) |
| 26 | 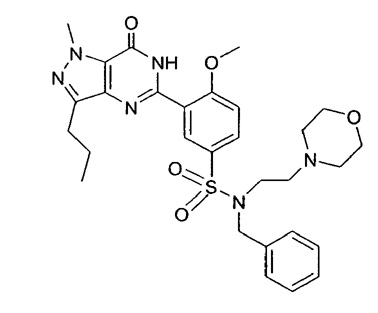 | 1-methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one 8.03 (1H, dd), 7.95 (1H, d), 7.32 (6H, m), 4.37 (2H, s), 4.15 (3H, s), 3.92 (3H, s), 3.86 (2H, t), 3.65 (2H, t), 3.53 (2H, t), 3.26 (2H, t), 3.09 (2H, t), 2.98 (2H, t), 2.77 (2H, t), 1.72 (2H, m), 0.92 (3H, t). (DMSO) |
| 27 | 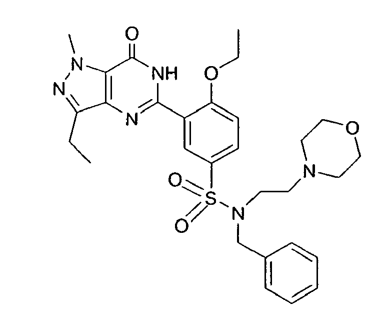 | 1-methyl-5-{2-ethoxy-5-[[1-benzyl-1-(2-morpholin-1-yl)ethyl]amidosulfonyl]phenyl}-3-ethyl-1,6-dihydro-7H-pyrazolo [4,3-d]pyrimidin-7-one 10.84 (1H, s), 8.94 (1H, d), 7.95 (1H, dd), 7.29 (5H, m), 7.14 (1H, d), 4.44 (2H, s), 4.28 (3H, s), 3.55 (2H, t), 3.28 (2H, t), 2.97 (2H, q), 2.36(2H, t), 2.25 (4H, t), 1.65 (3H, t), 1.25 (3H, t) |
| 28 | 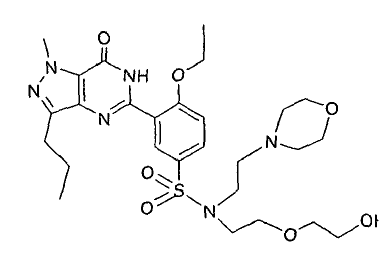 | 1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 8.84 (1H , d), 7.90 (1H, dd), 7.13 (1H, d), 4.37 (2H, m), 4.27 (3H, s), 3.65 (8H, m), 3.49 (4H, m), 3.43 (2H, t), 2.94 (2H, t), 2.64 (4H, d), 2.46 (2H, t), 1.85 (2H, m), 1.64 (3H, t), 1.01 (3H, t) (DMSO). |
| 29 | 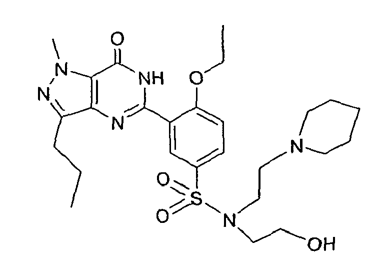 | 1-methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one 10.84(1H, s), 8.86(1H, d), 7.89(1H, dd), 7.15(1H, d), 4.37(2H, q), 4.27(3H, s), 3.68(2H, t), 3.39(2H, t), 2.93(2H, t), 2.69(2H, t), 2.49(2H, t), 2.41 (4H, br s), 1.85(2H, m), 1.64(3H, t), 1.62(4H, m), 1.44(2H, m), 1.02(3H, t) |
| 35 | 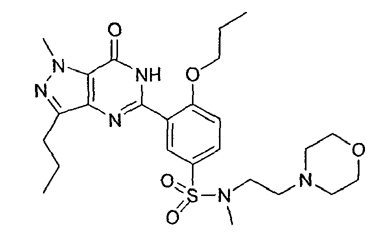 | 1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one 10.86 (1H, s), 8.86 (1H,d), 7.89 (1 H, dd), 7.15 (1H, d), 4.27 (3H, s), 4.25 (2H, t), 3.67 (4H, t), 3.21 (2H, t), 2.91 (2H, m), 2.87 (3H, s), 2.58 (2H, t), 2.47 (4H, t), 2.03 (2H, m), 1.84 (2H, m), 1.18 (3H, t), 1.02 (3H, t) |
| 36 | 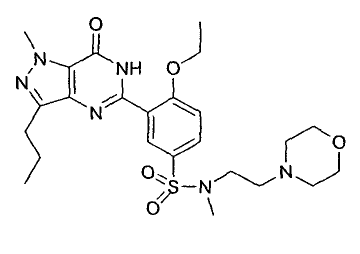 | 1-methyl-5-{2-ethoxy-5-[[1-methyl-1-(2-morpbolin-1-yl)ethyl]amidosulfonyl]phenyl}-3-ethyl-1,6-dihydro-7H-pyrazolo[4,3-d]pyrimidin-7-one 10.82 (1H, s), 8.85 5 (1H,d), 7.89 (1H, dd), 7.14 (1H, d), 4.37 (2H, q), 4.27 (3H, s), 3.68 (4H, t), 3.22 (2H, t), 2.92 (2H, t), 2.87 (3H, s), 2.58 (2H, t), 2.48 (4H, t), 1.84 (2H, m), 1.64 (3H, t), 1.02 (3H, t) |
| 41 | 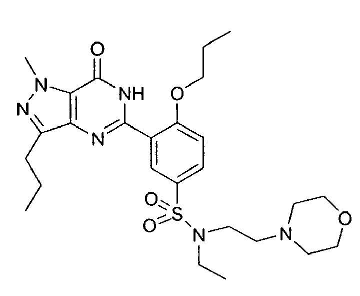 | 1-methyl-5-{2-propoxy-5-[[1-ethyl-1-(2-morpholin-1-yl)ethyl]amidosulfonyl]phenyl}-3-propyl-1,6-dihydro-7H-pyrazolo[4,3-d]pyrimidin-7-one 10.88 (1H, s), 8.89 (1H,d), 7.92 (1 H, dd), 7.12 (1 H, d), 4.27 (3H, s), 4.25 (2H, t), 3.65 (4H, t), 3.33 (4H, m), 2.92 (2H, t), 2.58 (2H, t), 2.47 (4H, t), 2.03(2H, m), 1.85 (2H, m), 1.19 (3H, t), 1.18 (3H, t), 1.02 (3H, t) |
| 43 | 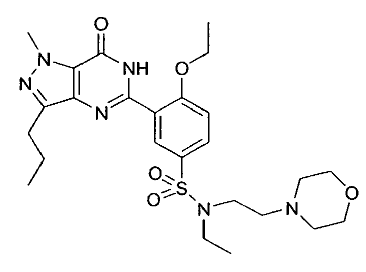 | 1-methyl-5-{2-ethoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one 10.84 (1H, s), 8.88 (1H,d), 7.92 (1H, dd), 7.12 (1H, d), 4.36 (2H, q), 4.27 (3H, s), 3.65 (4H, t), 3.33 (4H, m), 2.92 (2H, t), 2.58 (2H, t), 2.46 (4H, t), 1.85 (2H, m), 1.63 (3H, t), 1.18 (3H, t), 1.02 (3H, t) |
| 48 | 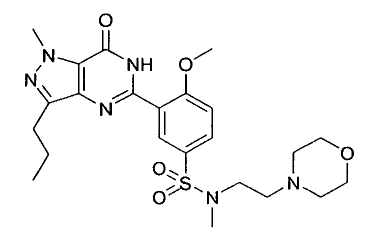 | 1-methyl-5-{2-methoxy-5-[[1-methyl-1-(2-morpholin-1-yl)ethyl]amidosulfonyl]phenyl}-3-n-propyl-1,6-dihydro-7H-pyrazolo[4,3-d]pyrimidin-7-one 10.63 (1 H, s), 8.84 (1H,d), 7.91 (1H, dd), 7.17 (1H, d), 4.27 (3H, s), 4.13 (3H, s), 3.67 (4H, t), 3.22 (2H, t), 2.92 (2H, t), 2.87 (3H, s), 2.58 (2H, t), 2.48 (4H, t), 1.85 (2H, m), 1.01 (3H, t) |
| 49 | 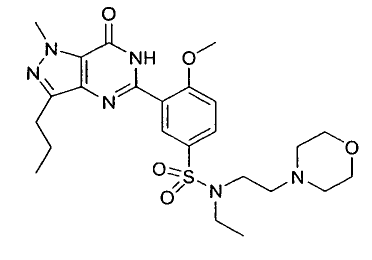 | 1-methyl-5-{2-methoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one 10.65 (1H, s), 8.88 (1H,d), 7.95 (1H, dd), 7.15 (1H, d), 4.27 (3H, s), 4.12 (3H, s), 3.65 (4H, t), 3.32 (4H, m), 2.92 (2H, t), 2.58 (2H, t), 2.46 (4H, t), 1.85 (2H, m), 1.19 (3H, t), 1.01 (3H, t) |
| 50 | 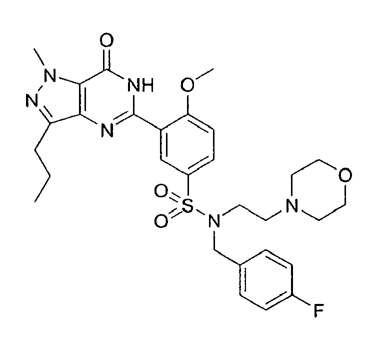 | 1-methyl-5-{2-methoxy-5-[[1-(4-fluorobenzyl)-1-(2-morpholin-l-y1)ethyl]amidosulfonyl]phenyl}-3-n-propyl-1,6-dihydro-7H-pyrazolo[4,3-d]pyrimidin-7-one 10.65 (1 H, s), 8.91 (1H, d), 7.96 (1H, dd), 7.31 (2H, m), 7.16 (1H, d), 7.01 (2H, m), 4.41 (2H, s), 4.28 (3H, s), 4.14 (3H, s), 3.56 (4H, t), 3.27 (2H, t), 2.91 (2H, t), 2.33 (2H, t), 2.26 (4H, t), 1.84 (2H, m), 0.99 (3H, t). |
| 53 | 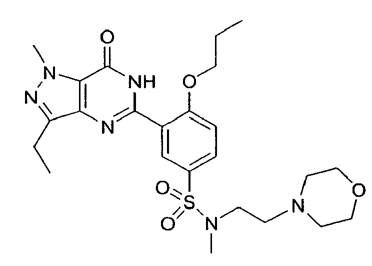 | 1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one 10.63 (1H, s), 8.86 (1H,d), 7.89 (1H, dd), 7.15 (1H, d), 4.27 (3H, s), 4.26 (2H, t), 3.70 (4H, t), 3.26 (2H, t), 2.97 (2H, q), 2.87 (3H, s), 2.63 (2H, t), 2.54 (4H, t), 2.04 (2H, m), 1.41 (3H, t), 1.18 (3H, t) |
| 54 | 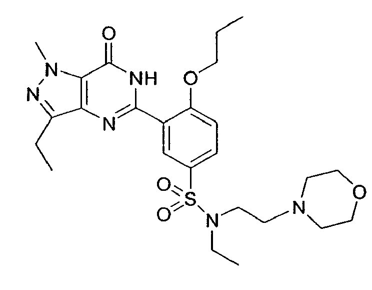 | 1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one 10.88 (1H, s), 8.90 (1H,d), 7.92 (1H, dd), 7.13 (1H, d), 4.27 (3H, s), 4.25 (2H, t), 3.69 (4H, t), 3.34 (4H, m), 2.97 (2H, q), 2.63 (2H, t), 2.52 (4H, t), 2.04 (2H, m), 1.41 (3H, t), 1.19 (3H, t), 1.18 (3H, t) |
| 55 | 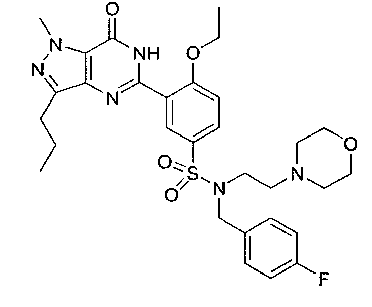 | 1-methyl-5-{2-ethoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one 10.83 (1H, s), 8.92 (1H, d), 7.94 (1H, dd), 7.31 (2H, m), 7.14 (1H, d), 7.01 (2H, m), 4.42 (2H, s), 4.37 (2H, q), 4.28 (3H, s), 3.56 (4H, t), 3.27 (2H, t), 2.92 (2H, t), 2.33 (2H, t), 2.26 (4H, t), 1.85 (2H, m), 1.65 (3H, t), 1.00 (3H, t). |
| 56 | 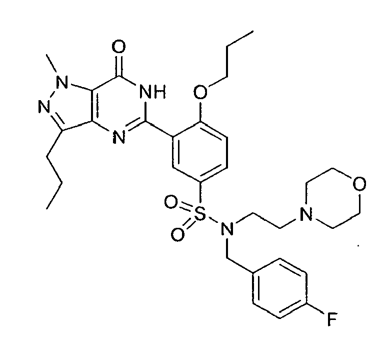 | 1-methyl-5-{2-propoxy-5-[[1-(4-fluorobenzyl)-1-(2-morpholin-1-yl)ethyl]amidosulibnyl]phenyl}-3-n-propyl-1,6-dihydro-7H-pyrazolo[4,3-d]pyrimidin-7-one 10.86 (1H, s), 8.92 (1H, d), 7.92 (1H, dd), 7.33 (2H, m), 7.16 (1H, d), 7.02 (2H, m), 4.41 (2H, s), 4.28 (3H, s), 4.27 (2H, t), 3.61 (4H, t), 3.31 (2H, t), 2.92 (2H, t), 2.39 (2H, t), 2.31 (4H, t), 2.05 (2H, m), 1.85 (2H, m), 1.19 (3H, t), 1.00 (3H, t). |
| 60 | 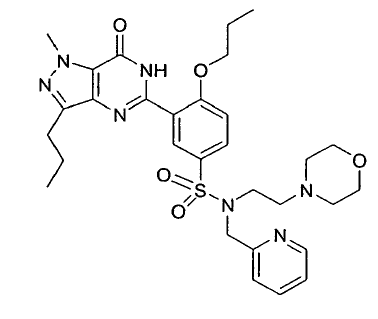 | 1-methyl-5-{2-propoxy-5-[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.87 (1H, s), 8.91 (1H, d), 7.92 (1H, dd), 8.45 (1H, d), 7.94 (1 H, d), 7.68 (1H, t), 7.60 (1H, d), 7.18 (1H, t), 7,16 (1H, d), 4.56 (2H, s), 4.28 (3H, s), 4.26 (2H, t), 3.58 (4H, t), 3.42 (2H, t), 2.92 (2H, t), 2.45 (2H, t), 2.34 (4H, t), 2.04 (2H, m), 1.85 (2H, m), 1.19 (3H, t), 1.01 (3H, t). |
| 61 | 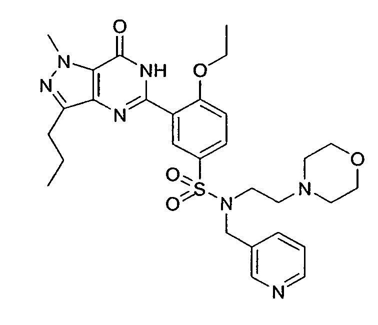 | 1-methyl-5-{2-ethoxy-5-[[1-(3-pyridylmethyl)-1-(2-morpholin-l-yl)ethyl]amidosulfonyl]phenyl}-3-n-propyl-1,6-dihydro-7H-pyrazolo[4,3-d]pyrimidin-7-one 10.84 (1H, s), 8.91 (1H, d), 8.54 (1H, dd), 8.48 (1H, d), 7.95 (1 H, dd), 7.84 (1H, d), 7.31 (1H, dd), 7.15 (1H, d), 4.48 (2H, s), 4.38 (2H, q), 4.28 (3H, s), 3.56 (4H, t), 3.32 (2H, t), 2.92 (2H, t), 2.38 (2H, t), 2.28 (4H, t), 1.85 (2H, m), 1.65 (3H, t), 1.00 (3H, t). |
| 62 | 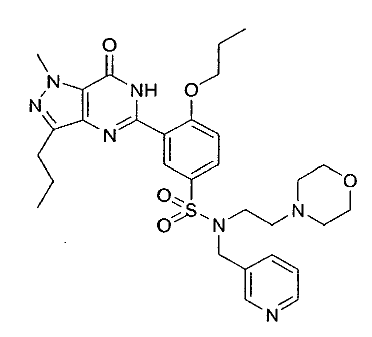 | 1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.8 8 (1H, s), 8.92 (1H, d), 8.54 (1H, d), 8.49 (1H, s), 7.94 (1H, dd), 7.82 (1H, d), 7.30(1H, dd), 7.16(1H, d), 4.47 (2H, s), 4.28 (3H, s), 4.27 (2H, t), 3.60 (4H, t), 3.34 (2H, t), 2.92 (2H, t), 2.41 (2H, t), 2.32 (4H, t), 2.05 (2H, m), 1.85 (2H, m), 1.19 (3H, t), 1.00 (3H, t). |
| 63 | 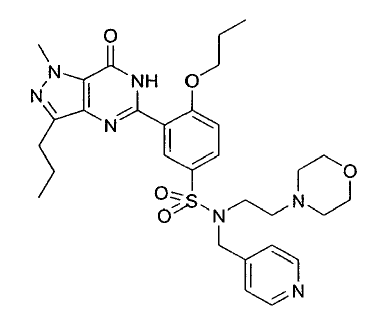 | 1-methyl-5-{2-propoxy-5-[[1-(4-pyridylmethyl)-1-(2-morpholin-1-yl)ethyl]amidosulfbnyl]phenyl}-3-n-propyl-1,6-dihydro-7H-pyrazolo[4,3-d]pyrimidin-7-one 10.87 (1H, s), 8.91 (1H, d), 8.56 (2H, d), 7.93 (1 H, dd), 7.29 (2H, d), 7.15(1H, d), 4.48 (2H, s), 4.27 (3H, s), 4.26 (2H, t), 3.55 (4H, t), 3.33 (2H, t), 2.92 (2H, t), 2.38 (2H, t), 2.26 (4H, t), 2.04 (2H, m), 1.86 (2H, m), 1.19 (3H, t), 1.01 (3H, t) |
| 64 | 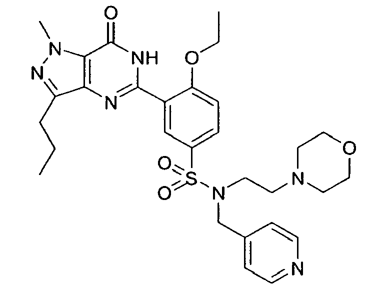 | 1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one 10.83 (1 H, s), 8.92 (1H, d), 8.56 (2H, dd), 7.93(1H, dd), 7.29(2H, d), 7.14 (1 H, d), 4.49 (2H, s), 4.38 (2H, q), 4.28 (3H, s), 3.55 (4H, t), 3.34 (2H, t), 2.92 (2H, t), 2.39 (2H, t), 2.26 (4H, t), 1.82 (2H, m), 1.65(3H, t), 1.01 (3H, t) |
| 71 | 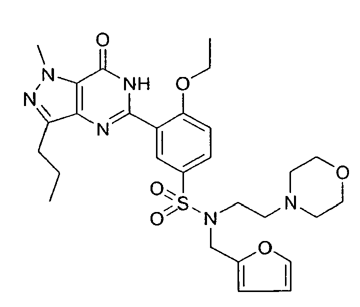 | 1-methyl-5-{2-ethoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7- one 10.80 (1H, s), 8.86 (1H, d), 7.86 (1H, dd), 7.25 (1H, m), 7.22 (1H, m), 7.07 (1H, d), 6.22 (2H, m), 4.55 (2H, s), 4.35 (2H, q), 4.27 (3H, s), 3.62 (4H, t), 3.34 (2H, t), 2.92 (2H, t), 2.53 (2H, t), 2.44 (4H, t), 1.85 (2H, m), 1.63 (3H, t), 1.00 (3H, t) |
| 72 | 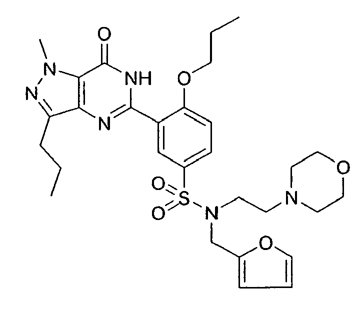 | 1-methyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one 10.82 (1H, s), 8.87 (1H, d), 7.86 (1H, dd), 7.25 (1H, m), 7.22 (1 H, m), 7.08 (1 H, d), 6.22 (2H, m), 4.55 (2H, s), 4.27 (3H, s), 4.24 (2H, t), 3.66 (4H, t), 3.33 (2H, t), 2.92 (2H, t), 2.53 (2H, t), 2.44 (4H, t), 2.02 (2H, m), 1.85 (2H, m), 1.17 (3H, t), 1.00 (3H, t) |
| 73 | 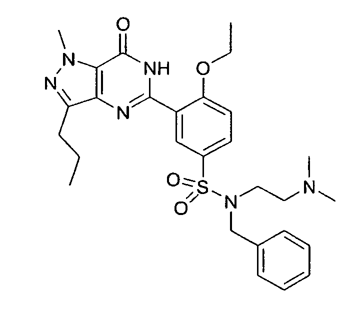 | 1-methyl-5-{2-ethoxy-5-[[1-benzyl-1-(2-dimethylaminoethyl)]amidosulfonyl]phenyl}-3-n-propyl-1,6-dihydro-7H-pyrazolo[4,3-d]pyrimidin-7-one 10.85 (1H, s), 8.94 (1H, d), 7.95 (1H, dd), 7.29-7.33 (5H, m), 7.14 (1H, d), 4.42 (2H, s), 4.38 (2H, q), 4.27 (s, 3H), 3.25 (2H, m), 2.92 (2H, t), 2.32 (2H, m), 2.08 (6H, s), 1.85 (2H, m), 1.65 (3H, t), 1.00 (3H, t) |
| 74 | 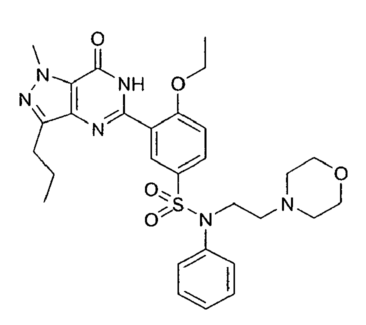 | 1-methyl-5-{2-ethoxy-5-[[1-phenyl-1-(2-morpholin-1-yl)ethyl]amidosulfonyl]phenyl}-3-ethyl-1,6-dihydro-7H-pyrazolo[4,3-d]pyrimidin-7-one 10.80 (1H, s), 8.71 (1H, d), 7.67 (1H, dd), 7.32 (3H, m), 7.15 (2H, m), 7.06 (1H, d), 4.36 (2H, q), 4.27 (3H, s), 3.76 (2H, t), 3.64 (4H, t), 2.88 (2H, t), 2.53 (2H, t), 2.44 (4H, t), 1.82 (2H, m), 1.64 (3H, t), 1.01 (3H, t) |
| 75 | 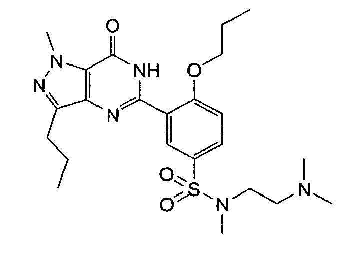 | 1-methyl-5-{2-propoxy-5-[[1-methyl-1-(2-dimethylaminoethyl)]amidosulfbnyl]phenyl}-3-n-propyl-1,6-dihydro-7H-pyrazolo[4,3-d]pyrimidin-7-one 10.85 (1H, s), 8.86 (1H, d), 7.88 (1H, dd), 7.16 (1H, d), 4.27 (s, 3H), 4.26 (2H, t), 3.29 (2H, t), 2.92 (2H, t), 2.87 (3H, s), 2.72 (2H, t), 2.40 (6H, s), 2.04 (2H, m), 1.85 (2H, m), 1.18 (3H, t), 1.01 (3H, t) |
| 76 | 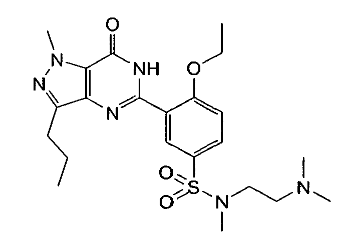 | 1-methyl-5-{2-ethoxy-5-[[1-methyl-1-(2-dimethylaminoethyl)]amidosulfonyl]phenyl}-3-n-propyl-1,6-dihydro-7H-pyrazolo[4,3-d]pyrimidin-7-one 10.82 (1H, s), 8.86 (1H, d), 7.88 (1H, dd), 7.16 (1H, d), 4.37 (2H, q), 4.27 (s, 3H), 3.22 (2H, t), 2.92 (2H, t), 2.86 (3H, s), 2.60 (2H, t), 2.32 (6H, s), 1.85 (2H, m), 1.64 (3H, t), 1.01 (3H, t) |
| 77 | 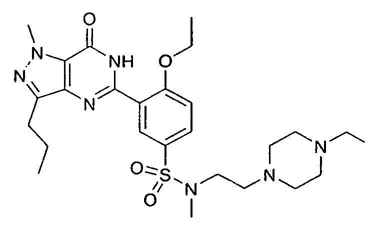 | 1-methyl-5-{2-ethoxy-5-[[1-methyl-1-[2-(4-ethylpiperazin-1-yl)]ethyl]amidosulfonyl]phenyl}-3-n-propyl-1,6-dihydro-7H-pyrazolo [4,3-d]pyrimidin-7-one 10.80 (1H, s), 8.85 (1H, d), 7.88 (1H, dd), 7.14 (1H, d), 4.37 (2H, q), 4.27 (s, 3H), 3.22 (2H, t), 2.92 (2H, t), 2.87 (3H, s), 2.58 (2H, t), 2.52 (4H, t), 2.44 (4H, t), 2.38 (2H, q), 1.85 (2H, m), 1.65 (3H, |
| 78 | 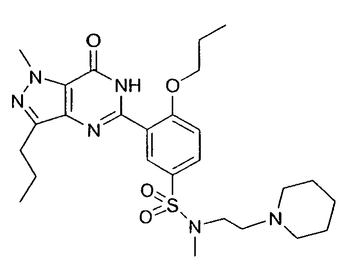 | t), 1.06 (3H, t), 1.02 (3H, t) 1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one 10.85 (1H, s), 8.86 (1H, d), 7.89 (1H, dd), 7.14 (1H, d), 4.27 (3H, s), 4.26 (2H, t), 3.22 (2H, t), 2.93 (2H, t), 2.86 (3H, s), 2.54 (2H, t), 2.40 (4H, t), 2.04 (2H, m), 1.85 (2H, m), 1.54 (4H, t), 1.41 (2H, m), 1.18 (3H, t), 1.02 (3H, t) |
| 79 | 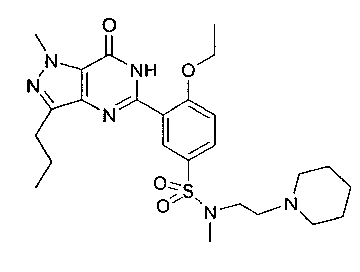 | 1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one 10.83 (1H, s), 8.85 (1H, d), 7.89 (1H, dd), 7.13(1H, d), 4.37 (2H, q), 4.27 (3H, s), 3.21 (2H, t), 2.93 (2H, t), 2.86 (3H, s), 2.54 (2H, t), 2.40 (4H, t), 1.86 (2H, m), 1.64 (3H, t), 1.53 (4H, t), 1.40 (2H, m), 1.02 (3H, t) |
| 80 | 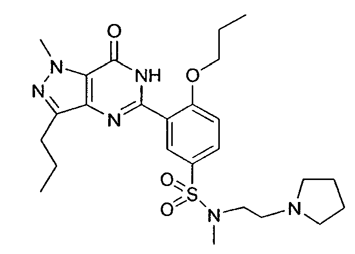 | 1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one 10.86 (1H, s), 8.87 (1H, d), 7.89 (1H, dd), 7.15 (1H, d), 4.27 (3H, s), 4.25 (2H, t), 3.22 (2H, t), 2.92 (2H, t), 2.86 (3H, s), 2.70 (2H, t), 2.54 (4H, m), 2.04 (2H, m), 1.86 (2H, m), 1.75 (4H, m), 1.18 (3H, t), 1.02 (3H, t) |
| 81 | 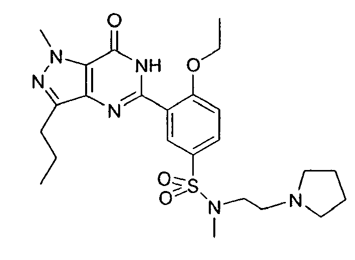 | 1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one 10.85 (1H, s), 8.85 (1H, d), 7.88 (1H, dd), 7.13 (1H, d), 4.37 (2H, q), 4.27 (3H, s), 3.22 (2H, t), 2.92 (2H, t), 2.86 (3H, s), 2.70 (2H, t), 2.53 (4H, m), 1.86 (2H, m), 1.75 (4H, m), 1.64 (3H, t), 1.02 (3H, t) |
| 82 | 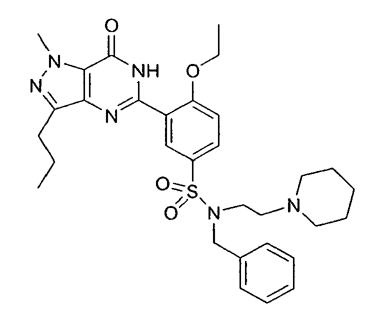 | 1-methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one 10.84 (1H, s), 8.93 (1H, d), 7.95 (1H, dd), 7.29-7.32 (5H, m), 7.13 (1H, d), 4.46 (2H, s), 4.38 (2H, q), 4.28 (3H, s), 3.30 (2H, t), 2.92 (2H, t), 2.33 (2H, t), 2.24 (4H, t), 1.85 (2H, m),1.65 (3H, t), 1.46 (4H, t), 1.35 (2H, m), 1.00 (3H, t) |
| 83 | 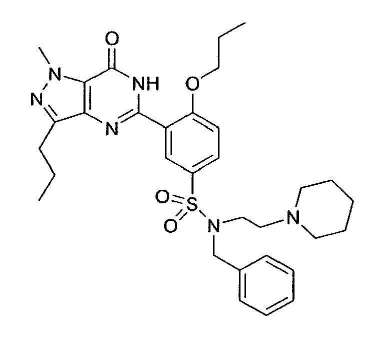 | 1-methyl-5-{2-propoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one 10.89 (1H, s), 8.94 (1H, d), 7.95 (1H, dd), 7.28-7.33 (5H, m), 7.14 (1H, d), 4.45 (2H, s), 4.28 (3H, s), 4.26 (2H, t), 3.33 (2H, t), 2.92 (2H, t), 2.38 (2H, t), 2.27 (4H, t), 2.05 (2H, m), 1.85 (2H, m), 1.50 (4H, t), 1.36 (2H, m), 1.19 (3H, t), 1.00 (3H, t) |
| 84 | 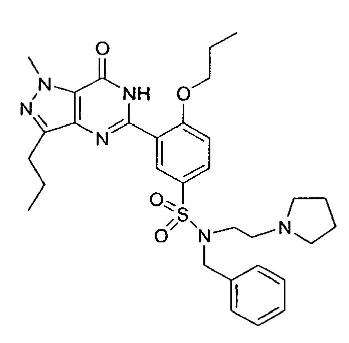 | 1-methyl-5-{2-propoxy-5-[[1-benzyl-1-(2-pyrrolidin-l-yl)ethyl]amidosulfonyl]phenyl}-3-n-propyl-1,6-dihydro-7H-pyrazolo[4,3-d]pyrimidin-7-one 10.89 (1H, s), 8.94 (1H, d), 7.95 (1H, dd), 7.30-7.34 (5H, m), 7.14 (1H, d), 4.42 (2H, s), 4.28 (3H, s), 4.27 (2H, t), 3.32 (2H, t), 2.92 (2H, t), 2.54 (2H, t), 2.45 (4H, s), 2.05 (2H, m), 1.85 (2H, m), 1.72 (4H, m), 1.19 (3H, t), 1.00 (3H, t) |
| 85 | 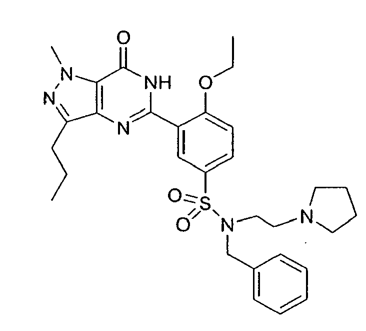 | 1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one 10.88 (1H, s), 8.93 (1H, d), 7.95 (1H, dd), 7.29-7.34 (5H, m), 7.14 (1H, d), 4.43 (2H, s), 4.38 (2H, q), 4.28 (3H, s), 3.32 (2H, t), 2.92 (2H, t), 2.55 (2H, t), 2.46 (4H, m), 1.85 (2H, m), 1.72 (4H, m), 1.65 (3H, t), 1.00 (3H, t) |
| 87 | 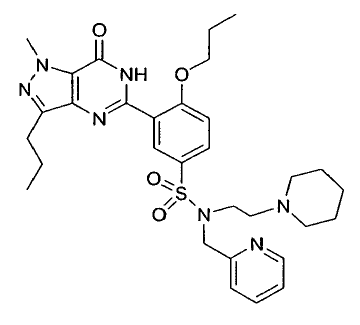 | 1-methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one 10.87 (1H, s), 8.91 (1H, d), 7.92 (1H, dd), 8.45 (1H, d), 7.95 (1H, d), 7.68 (1H, ddd), 7.57 (1H, d), 7.17 (1H, t), 7.13 (1H, d), 4.55 (2H, s), 4.28 (3H, s), 4.26 (2H, t), 3.45 (2H, t), 2.93 (2H, t), 2.47 (2H, t), 2.33 (4H, t), 2.05 (2H, m), 1.86 (2H, m), 1.50 (4H, t), 1.38 (2H, m), 1.19 (3H, t), 1.01 (3H, t) |
| 88 | 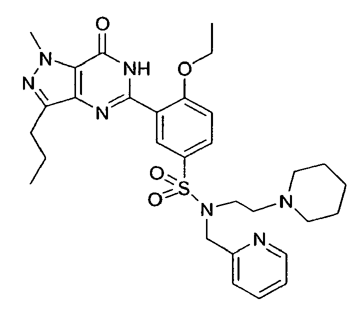 | 1-methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one 10.83 (1H, s), 8.90 (1H, d), 8.45 (1H, d), 7.95(1H, d), 7.68 (1H, ddd), 7.56 (1 H, d), 7.18 (1H, m), 7.13 (1H, dd), 4.56 (2H, s), 4.38 (2H, q), 4.28 (3H, s), 3.46 (2H, t), 2.92 (2H, t), 2.48 (2H, t), 2.34 (4H, t), 1.85 (2H, m), 1.65 (3H, t), 1.52 (4H, t), 1.38 (2H, m), 1.02 (3H, t) |
| 89 | 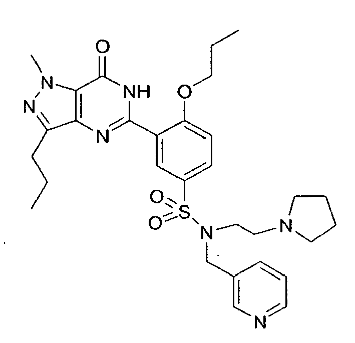 | 1-methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one 10.88 (1H, s), 8.93 (1H, d), 8.54 (1H, dd), 8.50 (1H, s), 7.94 (1H, dd), 7.81 (1H, d), 7.30 (1H, dd), 7.15 (1H, d), 4.46 (2H, s), 4.28 (3H, s), 4.27 (2H, t), 3.36 (2H, t), 2.92 (2H, t), 2.58 (2H, t), 2.48 (4H, t), 2.05 (2H, m), 1.85 (2H, m), 1.73 (4H, t), 1.19 (3H, t), 1.00 (3H, t) |
| 90 | 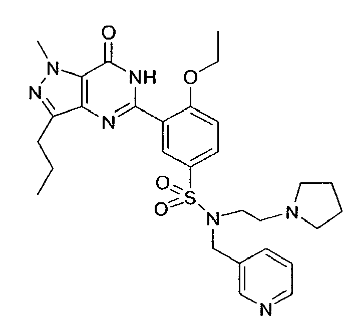 | 1-methyl-5-{2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one 10.85 (1H, s), 8.91 (1H, d), 8.54 (1H, dd), 8.50 (1H, d), 7.94 (1H, dd), 7.81 (1H, d), 7.30 (1H, dd), 7.15 (1H, d), 4.46 (2H, s), 4.38 (2H, q), 4.28 (3H, s), 3.37 (2H, t), 2.92 (2H, t), 2.60 (2H, t), 2.50 (4H, t), 1.85 (2H, m), 1.74 (4H, t), 1.65 (3H, t), 1.00 (3H, t) |
| 95 | 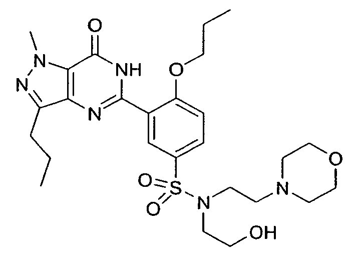 | 1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one 10.81 (1H, s), 8.85 (1H,d), 7.88 (1H, dd), 7.15 (1H, d), 4.27 (3H, s), 4.25 (2H, t), 3.83 (2H, t), 3.74 (4H, t), 3.28 (2H, t), 3.22 (2H, t), 2.92 (2H, t), 2.72 (2H, t), 2.58 (4H, t), 2.03 (2H, m), 1.85 (2H, m), 1.18 (3H, t), 1.02 (3H, t) |
| 96 | 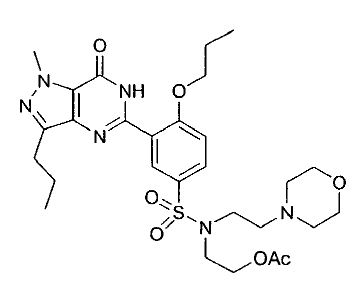 | 1-methyl-5-{2-propoxy-5-[[1-(2-acetoxyethyl)-1-(2-morpholin-1-yl)ethyl]amidosulfonyl]phenyl-3-n-propyl-1,6-dihydro-7H-pyrazolo[4,3-d]pyrimidin-7-one 10.84 (1H, s), 8.88 (1H,d), 7.91 (1 H, dd), 7.13 (1H, d), 4.27 (3H, s), 4.25 (2H, t), 4.23 (2H, t), 3.63 (4H, t), 3.48 (2H, t), 3.35 (2H, t), 2.92 (2H, t), 2.58 (2H, t), 2.44 (4H, t), 2.04 (2H, m), 2.02 (3H, s), 1.85 (2H, m), 1.17 (3H, t), 1.02 (3H, t) |
| 98 | 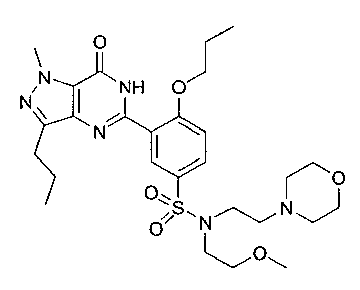 | 1-methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one 10.86 (1H, s), 8.89 (1H,d), 7.92 (1H, dd), 7.12 (1H, d), 4.27 (3H, s), 4.24 (2H, t), 3.64 (4H, t), 3.56 (2H, t), 3.43 (2H, t), 3.37 (2H, t), 3.29 (3H, s), 2.92 (2H, t), 2.60 (2H, t), 2.45 (4H, t), 2.03 (2H, m), 1.85 (2H, m), 1.18 (3H, t), 1.02 (3H, t) |
| 100 | 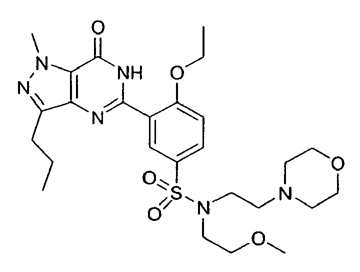 | 1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one 10.82 (1H, s), 8.88 (1H, d), 7.92 (1H, dd), 7.11 (1H, d), 4.36 (2H, q), 4.27(3H, s), 3.64 (4H, t), 3.55 (2H, t), 3.43 (2H, t), 3.37 (2H, t), 3.29 (3H, s), 2.92 (2H, t), 2.60 (2H, t), 2.45 (4H, t), 1.86 (2H, m), 1.63 (3H, t), 1.02(3H, t) |
| 104 | 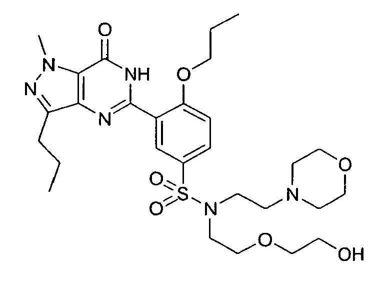 | 1-ethyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 10.85 (1H, s), 8.84 (1H , d), 7.90 (1H, dd), 7.13 (1H, d), 4.27 (3H, s), 4.25 (2H, t), 3.65 (8H, m), 3.49 (4H, t), 3.42 (2H, t), 2.94 (2H, t), 2.64 (2H, t), 2.44 (4H, t), 2.03 (2H, m), 1.85 (2H, m), 1.17 (3H, t), 1.01 (3H, t). |
| 105 | 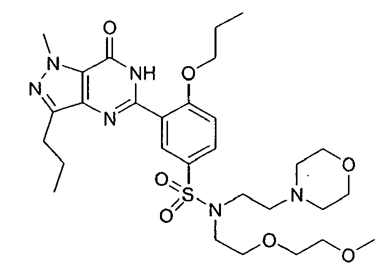 | 1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one 10.85 (1H, s), 8.88 (1H, d), 7.93 (1H, dd), 7.12 (1H, d), 4.27 (3H, s), 4.25 (2H, t), 3.67 (2H, t), 3.65 (4H, t), 3.57 (2H, t), 3.48 (2H, t), 3.43 (2H, t), 3.38 (2H, t), 3.33 (3H, s), 2.92 (2H, t), 2.62 (2H, t), 2.46 (4H, t), 2.03 (2H, m), 1.85 (2H, m), 1.18 (3H, t), 1.02 (3H, t) |
| 110 | 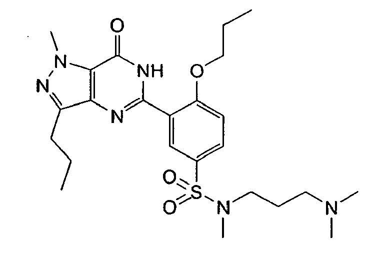 | 1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one 10.88 (1H, s), 8.88 (1H, d), 7.90 (1H, dd), 7.15 (1H, d), 4.30 (s, 3H), 4.27 (2H, t), 3.15 (2H, t), 2.95 (2H, t), 2.83 (3H, s), 2.43 (2H, t), 2.30 (6H, s), 2.06 (2H, m), 1.89 (2H, m), 1.80 (2H, m), 1.20 (3H, t), 1.05 (3H, t) |
| 111 | 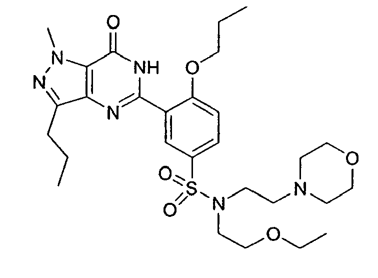 | 1-methyl-5-{2-propoxy-5-[[1-(2-ethoxyethyl)-1-(2-morpholin-1-yl)ethyl]amidosulfonyl]phenyl}-3-n-propyl-1,6-dihydro-7H-pyrazolo[4,3-d]pyrimidin-7-one 10.85 (1H, s), 8.88 (1H, d), 7.92 (1H, dd), 7.12 (1H, d), 4.27(3H, s), 4.24 (2H, t), 3.64 (4H, t), 3.59 (2H, t), 3.44 (2H, q), 3.42 (2H, t), 3.39 (2H, t), 2.92 (2H, t), 2.62 (2H, t), 2.46 (4H, t), 2.03 (2H, t), 1.85 (2H, m), 1.18 (3H, t),1.12 (3H, t), 1.02(3H, t) |
| 112 | 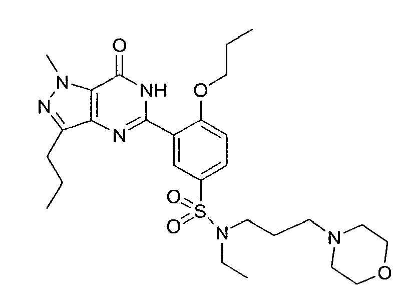 | 1-methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-one 10.86 (1H, s), 8.86 (1H, d), 7.89 (1H, dd), 7.12 (1H, d), 4.26 (3H, s), 4.24 (2H, t), 3.70 (4H, t), 3.30 (2H, q), 3.23 (2H, t), 2.92 (2H, t),2.45 (4H, t), 2.42 (2H, t), 2.03 (2H, m), 1.85 (4H, m), 1.17 (3H, t), 1.15 (3H, t), 1.01 (3H, t) |
| 113 | 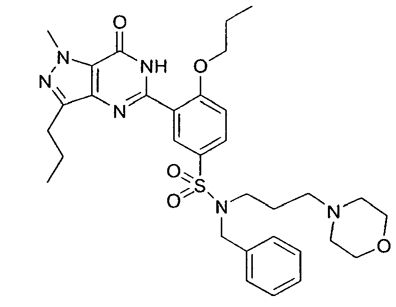 | 1-methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-one 10.87 (1H, s), 8.91 (1H, d), 7.91 (1H, dd), 7.27-7.32 (5H, m), 7.14 (1H, d), 4.37 (2H, s), 4.27 (3H, s), 4.26 (2H, t), 4.24 (2H, t), 3.57 (4H, t), 3.20 (2H, t), 2.92 (2H, t), 2.19 (4H, t), 2.04 (2H, m), 1.84 (2H, m), 1.58 (2H, m), 1.18 (3H, t), 0.99 (3H, t) |
| 117 | 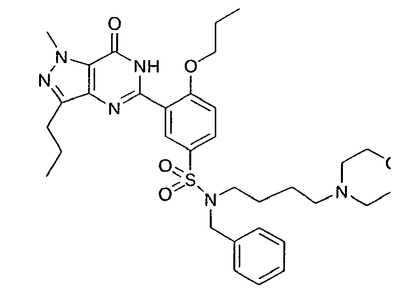 | 1-methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-one 10.88 (1H, s), 8.91 (1H, d), 7.91 (1H, dd), 7.27-7.30 (5H, m), 7.14 (1H, d), 4.37 (2H, s), 4.27 (3H, s), 4.25 (2H, t), 3.62 (4H, t), 3.17 (2H, t), 2.92 (2H, t), 2.30 (4H, t), 2.15 (2H, t), 2.04 (2H, m), 1.84 (2H, m), 1.35 (4H, m), 1.18 (3H, t), 0.99 (3H, t) |
| 119 | 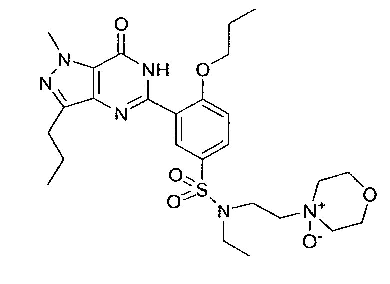 | 1-ethyl-5-{2-propoxy-5-[[1-ethyl-1-[2-(morpholin-N-oxide)-1-yl)ethyl]amidosulfonyl]phenyl}-3-n-propyl-1,6-dihydro-7H-pyrazolo[4,3-d]pyrimidin-7-one 10.87 (1H, s), 8.86 (1H,d), 7.89 (1H, dd), 7.17 (1H, d), 4.36 (2H, m), 4.28 (3H, s), 4.26 (2H, t), 3.84 (2H, d), 3.77 (4H, m), 3.45 (4H, m), 3.35 (2H, q), 2.92 (2H, t), 2.03 (2H, m), 1.85 (2H, m), 1.22 (3H, t), 1.18 (3H, t), 1.01 (3H, t) |
| 120 | 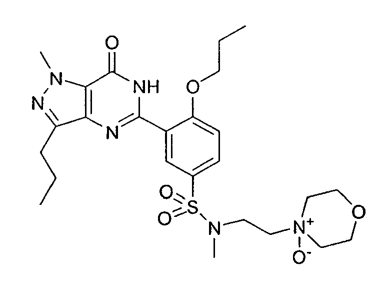 | 1-methyl-5-{2-propoxy-5-[[1-methyl-1-[2-(morpliolin-N-oxide)-1-yl)ethyl]amidosulfonyl]phenyl}-3-n-propyl-1,6-dihydro-7H-pyrazolo[4,3-d]pyrimidin-7-one 10.88 (1H, s), 8.88 (1H,d), 7.87 (1H, dd), 7.17 (1H, d), 4.41 (2H, t), 4.27 (3H, s), 4.26 (2H, t), 3.80 (2H, d), 3.70 (2H, t), 3.60 (2H, t), 3.43 (2H, t), 3.23 (2H, t), 2.92 (2H, t), 2.03 (2H, m), 1.85 (2H, m), 1.18 (3H, t), 1.01 (3H, t) |

### Example 121: Capsules

| Formulation | Quantity/1000 Capsules |
|---|---|
| Active ingredient (Pyrazolopyrimidinone derivatives) | 20g |
| Starch | 80g |
| Lactose | 60g |
| Microcrystalline cellulose | 35g |
| 10% Polyvinylpyrrolidone ethanol solution | q.s. |
| Magnesium stearate | 0.5g |
| Total 1000 Capsules | |

The active ingredient containing pyrazolopyrimidinone derivatives and the excipients are passed through a #80 mesh sieve, weigh out the appropriate amount of active ingredient and the excipients according the formulation. Granulate the powder mixture with 10% polyvinylpyrrolidone ethanol solution, and pass through a #16 mesh sieve to obtain suitable granules. After drying at 65°C, the granules were screened through a #14 sieve and blended with the magnesium stearate. Test the content of active ingredient in the granules, calculate the fill weight, and then fill the granules in capsules.

### Example 122 Tablets (wet granulation)

| Formulation | Quantity/1000 Tablets |
|---|---|
| Active ingredient (Pyrazolopyrimidinone derivatives) | 20g |
| Lactose | 120g |
| Microcrystalline cellulose | 40g |
| 8% Starch paste | q.s. |
| Sodium starch glycolate | 10g |
| Magnesium stearate | 1.0g |
| Total 1000 Tablets | |

Pass the active ingredient containing pyrazolopyrimidinone derivatives, microcrystalline cellulose, lactose, sodium starch glycolate through #80 mesh sieve, mix well. Granulate the powder mixture with 8% starch paste, and pass through a #16 mesh sieve to obtain suitable granules. After drying, the granules were screened and blended with the magnesium stearate. Test the content of active ingredient in the granules, calculate the tablet weight, and then compress the tablets.

### Example 123: Tablets (Direct Compression)

| Formulation | Quantity/1000 Tablets |
|---|---|
| Active ingredient (Pyrazolopyrimidinone derivative) | 20.0g |
| Microcrystalline cellulose | 30.0g |
| Lactose, anhydrous | 45.0g |
| Polyvinyl pyrrolidone | 3.0g |
| Aerosil | 0.2g |
| Magnesium stearate | 0.5g |
| Total 1000 Tablets | |

Charge the active ingredient containing pyrazolopyrimidinone derivatives, lactose, polyvinyl pyrrolidone, aerosol in a mixer and mix well. Blend the mixture with magnesium stearate and then compress into tablets.

### Test 1: pharmacodynamic test

The test was carried out based on the methods reported (International Journal of Impotence Research 2002, 14, 251 and The Journal of Urology 1992, 147, 1124). After fasted for 12 hours, 4 male SD rats were randomized into each group. After anesthetizing the rats with sodium pentobarbital (50 mg/kg, i.p.), the penile skin was incised and the prepuce was degloved to expose completely the corpora cavemosa (CC). A needle linked to an electrophysiology instrument was inserted into the CC on the right side in order to measure the intracavernous pressure (ICP). The right carotid artery was cannulated in a similar manner to the polyethylene tube in order to monitor the mean blood pressure (MBp) continuously. After exposing the lateral surface of the prostate via an incision in the midline inferior abdomen, a bipolar platinum microelectrode was placed on the cavernous nerve. Electric stimulation was performed at 2 Hz, for 60 s with a pulse duration of 5ms and 3V using a stimulator. The compounds were administrated orally (5mg/kg). The change of the ICP and MBp were monitored continuously before and after the administration. The effect of the compounds on the erection induced by electric stimulation was evaluated by the ratio of ICP to MBp. The parameter (ICP/MBp) was used to estimate the influence of the compounds to the rat corpora cavemosa. We tested the effect of sildenafil and some of the example compounds on the rat corpora cavemosa according to the aforesaid method. The statistical significance of the differences between groups was calculated using Duncan's multiple comparison. The results are shown below:

| Test compound | ICP/BP |
|---|---|
| blank | 27.5±2.3 |
| sildenafil | 80.1±5.1*** |
| 54 | 86.3±6.4*** |
| 62 | 69.8±12.2*** |
| 75 | 74.2±7.8*** |
| 78 | 71.6±8.3*** |
| 89 | 70.9±9.8*** |
| 95 | 57.2±10.1* |
| 96 | 81.0±9.5*** |
| 111 | 89.1±6.9*** |

| | |
|---|---|
| Note: Compared with the blank group , *P<0.05, ***P<0.001 | |

As shown in the results, the test compounds have the same pharmacodynamic effect as sildenafil. After administration, the ICP of the rats CC, and the ICP/BP increase significantly, the penile erections of the rats are enhanced, thus, the compounds can be administrated orally for the treatment of erectile dysfunction.

### Test 2: Enzyme inhibitory activity test

The Enzymes used in the inhibitory activity test were isolated from different kinds of tissues after appropriate treatment by FPLC using a method similar to Thrombosis Res. 1991, 612, 31 and J. Biol. Chem. 1997, 272, 2714. The PDE5 and PDE3 were isolated form human platelets, while the PDE6 was isolated form bovine retinas. The enzyme inhibitory activity test conducted immediately after the enzymes had been isolated, using a scintillation proximity assay for the direct detection of AMP/GMP by the TRKQ7100 and TRKQ7090 kit. In summary, the effect of PDE inhibitors was investigated by assaying a fixed amount of enzyme in the presence of varying inhibitor concentrations and low substrate. The final assay volume was made up to 100µl with 10µl assay buffer (50mM Tris/HCl PH 7.5, 8.3mM MgCl2, 1.7mM EGTA), and water. Reactions were initiated with enzyme, incubate for 30 minutes at 30°C and terminated with 50µl yttrium silicate SPA beads suspension containing zinc sulphate. Shook for 20 minutes and settled for 30 minutes in the dark, then counted on a BECKMAN LS6500 MULTIPURPOSE SCINTILLATION COUNTER. The IC50 value for the compounds according the present invention was calculated according the counts.

### PDE5 inhibitory activity test

According to the above-mentioned method, the inhibitory activities of some compounds of formula 1B according to the invention against PDE5 from human paletelets were determined. The result is given in the following table:

| Test compound | PDE5 IC₅₀ (nM) | Test compound | PDE5 IC₅₀ (nM) |
|---|---|---|---|
| sildenafil | 15.7 | 24 | 0.087 |
| 1 | 0.080 | 25 | 0.335 |
| 2 | 0.133 | 26 | 24.9 |
| 11 | 38.1 | 27 | 0.456 |
| 12 | 6.98 | 28 | 0.681 |
| 13 | 7.37 | 29 | 0.310 |
| 14 | 0.862 | 41 | 10.47 |
| 15 | 10.6 | 54 | 9.81 |
| 16 | 13.4 | 78 | 8.72 |
| 17 | 8.55 | 84 | 11.72 |
| 18 | 6.32 | 87 | 8.77 |
| 19 | 4.29 | 89 | 7.85 |
| 20 | 0.505 | 96 | 4.24 |
| 21 | 0.928 | 104 | 13.08 |
| 22 | 0.294 | 111 | 6.83 |
| 23 | 0.072 | 112 | 12.08 |

The IC₅₀ values for the compounds in the previous table show that most of the compounds according to the invention have a stronger potency against PDE5 than sildenafil, therefore, the dosage for oral administration is less than sildenafil and the chance to induce side effects is relatively little.

### PDE6 inhibitory activity test

Considering the compounds according the invention may have inhibitory activity against PDE6 distributed in retina, and then lead to visual disorders, we tested the inhibitory activities of some of the compounds of formulae 1B according the invention against PDE6 from bovine retina. The result is given in the following table:

| Test compound | PDE6 IC₅₀ (nM) | PDES IC₅₀ (nM) | PDE6 IC₅₀ / PDE5 IC₅₀ |
|---|---|---|---|
| sildenafil | 195.6 | 15.7 | 12.4 |
| 1 | 16.8 | 0.080 | 210 |
| 2 | 56.5 | 0.133 | 425 |
| 24 | 5.71 | 0.087 | 65.6 |
| 25 | 93.6 | 0.335 | 279 |
| 54 | 234.7 | 9.81 | 23.9 |
| 89 | 215.2 | 7.85 | 27.4 |
| 96 | 244.5 | 4.24 | 57.7 |

The invention uses the value of IC₅₀ PDE6/ IC₅₀ PDE5 to estimate the selectivity of PDE5 versus PDE6. The results show that most of the example compounds have a better selectivity than sildenafil, thus, the chance of visual disorder induced by the compounds according to the invention is less than sildenafil.

### PDE3 inhibitory activity test

PDE3 is a PDE isozyme distributed mainly in heart, so the inhibiting of PDE3 may lead to side effects associated with heart. Accordingly, the inhibitory activities of some example compounds according to the invention against PDE3 were determined. The result is given in the following table:

| Test compound | PDE3 IC₅₀ (µM) | Test compound | PDE3 IC₅₀ (µM) |
|---|---|---|---|
| sildenafil | 7.31 | 96 | 12.41 |
| 54 | 16.27 | 111 | 8.73 |
| 62 | 2.86 | | |

As shown in the table, since the 50% inhibition concentration (IC₅₀) for PDE 3 is much higher than for PDE5 in some of the compounds of the pyrazolopyrimidinone derivatives, the probability of side effects in cardiovascular system caused by the compounds of the present invention is very little.

### Test 3: Acute Oarl Toxicity Test

In this test male KM mouse weighting 18-22g were used, and 10 or 11 mouse were assigned randomly to each group. The compounds of examples 23, 33, 35, 37, 41, 54, 62, 63, 89, 92, 93, 95, 96, 97, 99, 103, 104, 110, 111, 112, 118 and sildenafil were suspended in 0.5% sodium carboxymethylcellulose respectively, and administered orally with single dose of 3g/kg. The animals were fasted for 12 hours before the administration. After the administration, the animals were observed for clinical signs of toxicity or mortality. The results were shown in the following table:

| Test compound | Number of mouse | Number of death | mortality rate (%) |
|---|---|---|---|
| sildenafil | 10 | 6 | 60 |
| 23 | 10 | 0 | 0 |
| 35 | 10 | 0 | 0 |
| 41 | 10 | 0 | 0 |
| 54 | 10 | 0 | 0 |
| 62 | 10 | 6 | 60 |
| 63 | 11 | 1 | 9 |
| 89 | 11 | 5 | 45 |
| 95 | 10 | 1 | 10 |
| 96 | 10 | 0 | 0 |
| 111 | 10 | 0 | 0 |
| 112 | 10 | 2 | 20 |
| 118 | 10 | 0 | 0 |

There were no significant clinical symptoms, weight changes and mortalities during the test. The results of autopsy in dead animals show that no abnormal signals such as bleeding of the internal organs were found. The results show that the toxicities of most of the compounds according to the present invention to mouse are significantly lower than sildenafil.

## Claims

1. A compound of formula (1B), or a pharmaceutically acceptable salt or solvate thereof, 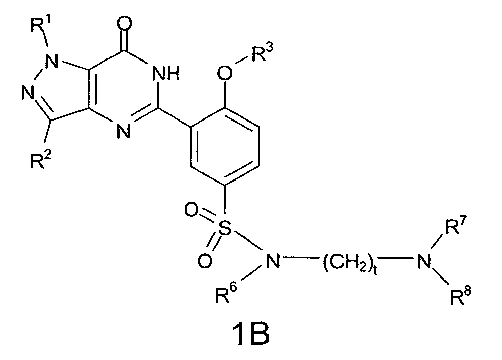 wherein
R¹ represents H, C₁-C₆ alkyl, C₃-C₆ cycloalkyl, C₁-C₃ alkyl substituted by halogen, or C₁-C₃ alkyl substituted by C₃-C₆ cycloalkyl;
R² represents C2-C6 alkyl, C₃-C₆ cycloalkyl, C₁-C₃ alkyl substituted by halogen, or C₁-C₃ alkyl substituted by C₃-C₆ cycloalkyl;
R³ represents C₁-C₆ alkyl, C₃-C₆ cycloalkyl, C₁-C₃ alkyl substituted by halogen, C₁-C₃ alkyl substituted by C₁-C₃ alkoxyl, or C₁-C₃ alkyl substituted by C₃-C₆ cycloalkyl;
t=1-5;
R⁶ represents C₁-C₆ alkyl, C₃-C₆ cycloalkyl, C₁- C₃ haloalkyl, C₁- C₃ alkoxyl, phenyl, pyridyl, furanyl, pyridazinyl, pyrazinyl, imidazolyl, C₁-C₃ substituted with hydroxyl, C₁-C₃ alkoxyl, acetoxyl, phenyl, pyridyl, furanyl, pyridazinyl, pyrimidinyl, pyrazinyl, imidazolyl; the above phenyl, pyridyl, furanyl, pyridazinyl, pyrimidinyl, pyrazinyl, imidazolyl optionally substituted with one or more substituents selected from halogen, C₁- C₃ alkyl, C₁- C₃ alkoxyl;
R⁷ and R⁸ independently represent H, C₁-C₆ alkyl, C₃-C₆ cycloalkyl, C₁-C₃ haloalkyl, C₁-C₆ alkoxyl, C₁- C₃ alkyl substituted with hydroxyl, acetoxyl, C₁-C₃ alkoxyl, or R⁷ and R⁸ together with the nitrogen to which they are attached form a four-membered to eight-membered heterocyclic ring, including morpholine, piperidine, pyrrole, piperazine; the above heterocyclic ring optionally substituted with one or more substituents selected from halogen, C₁- C₃ alkyl, C₃-C₆ cycloalkyl, C₁-C₃ haloalkyl, C₁- C₃ alkoxyl.

2. The compound according to claim 1,
wherein
R¹ represents C₁-C₄ alkyl or C₃-C₆ cycloalkyl;
R² represents C₂-C₄ alkyl or C3-C6 cycloalkyl;
R³ represents C₁-C₃ alkyl, C₁-C₃ alkyl substituted with C₁-C₃ alkoxyl;
t=2-3;
R⁶ represents C₁-C₃ alkyl, phenyl, pyridyl, or C₁-C₃ substituted with hydroxyl, C₁-C₃ alkoxyl, acetoxyl, phenyl, pyridyl;
R⁷ and R⁸ together with the nitrogen to which they are attached form a morpholine, piperidine or pyrrole heterocyclic ring.

3. The compound according to claim 2,
wherein
R¹ represents methyl or ethyl;
R² represents ethyl and n-propyl;
R³ represents ethyl, n-propyl or methyloxyethyl;
t=2-3,
R⁶ represents methyl, ethyl, benzyl, pyridylmethyl, or C₁-C₃ substituted with hydroxyl, C₁-C₃ alkoxyl, acetoxyl;
R⁷ and R⁸ together with the nitrogen to which they are attached form a morpholine, piperidine or pyrrole heterocyclic ring.

4. The compound according to claim 1, which is selected from the group consisting of:
1-methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 13)
1-methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-one (compound of example 14)
1-methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-one (compound of example 16)
1-ethyl-5-{2-ethoxy-5-{[1-ethyl-1-[2-(1-ethyl-1-(2-acetoxyethyl))amino]ethyl]amidosulfonyl}phenyl}-3-n-propyl-1,6-dihydro-7H-pyrazolo[4,3-d]pyrimidin-7-one (compound of example 19)
1-methyl-5-{2-ethoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 21)
1-methyl-5-{2-propoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-one (compound of example 23)
1-methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 24)
1-methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-one (compound of example 25)
1-methyl-5-{2-methoxy-5-[[1-benzyl-1-(2-morpholil1-1-yl)ethyl]amidosulfonyl]phenyl}-3-n-propyl-1,6-dihydro-7H-pyrazolo[4,3-d]pyrimidin-7-one (compound of example 26)
1-methyl-5-{2-ethoxy-5-[[1-benzyl-1-(2-morpholin-1-yl)ethyl]amidosulfonyl]phenyl}-3-ethyl-1,6-dihydro-7H-pyrazolo[4,3-d]pyrimidin-7-one (compound of example 27)
1-methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 28)
1-methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-one (compound of example 29)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 35)
1-methyl-5-{2-ethoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 36)
1-methyl-5-{2-propoxy-5-[[1-ethyl-1-(2-morpholin-1-yl)ethyl]amidosulfonyl]phenyl}-3-propyl-1,6-dihydro-7H-pyrazolo[4,3-d]pyrimidin-7-one (compound of example 41)
1-methyl-5-{2-cthoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-one (compound of example 43)
1-methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-one (compound of example 53)
1-methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-one (compound of example 54)
1-methyl-5-{2-propoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-one (compound of example 56)
1-methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 60)
1-methyl-5-{2-ethoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 61)
1-methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 62)
1-methyl-5-{2-propoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 63)
1-methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-one (compound of example 64)
1-methyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-one (compound of example 72)
1-methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one(compound of example 78)
1-methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-one(compound of example 79)
1-methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one(compound of example 80)
1-methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-one(compound of example 81)
1-methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one(compound of example 82)
1-methyl-5-{2-propoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-one(compound of example 83)
1-inethyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one(compound of example 84)
1-methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-one(compound of example 85)
1-methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 87)
1-methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-one (compound of example 88)
1-methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 89)
1-methyl-5-{2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-one (compound of example 90)
1-methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 95)
1-methyl-5-{2-propoxy-5-[[1-(2-acetoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 96)
1-methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 98)
1-methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 100)
1-methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 104)
1-methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 105)
1-methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-one (compound of example 110)
1-methyl-5-{2-propoxy-5-[[1-(2-ethoxyethyl)-1-(2-morpholin-1-yl)ethyl]amidosulfonyl]phenyl}-3-n-propyl-1,6-dihydro-7H-pyrazolo[4,3-d]pyrimidin-7-one (compound of example 111)
1-methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-one (compound of example 112)
1-methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-one (compound of example 113)
1-methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-one (compound of example 117).

5. A pharmaceutical composition comprising a compound of formula 1B as defined in any one of claims 1 to 4, or a pharmaceutically acceptable salt or solvate thereof, together with one or more pharmaceutically acceptable excipients.

6. The composition according to claim 5, the effective dosage is 1-500mg/day, preferably 10-100mg/day.

7. A process for the preparation of the compounds of formula 1B according to claim 1, or a prodrug thereof, or a pharmaceutically acceptable salt or solvate thereof, wherein the compounds of formula 1 B are prepared according to the following scheme: 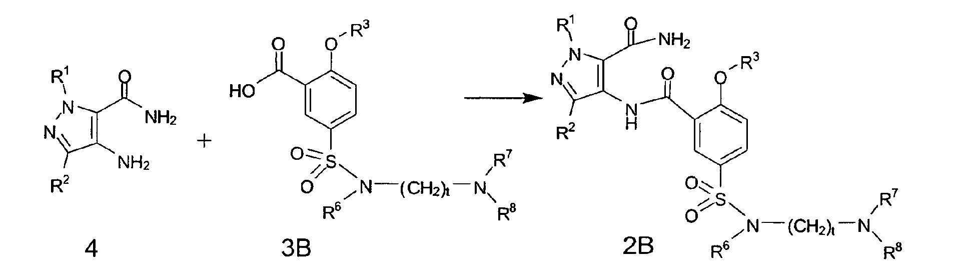 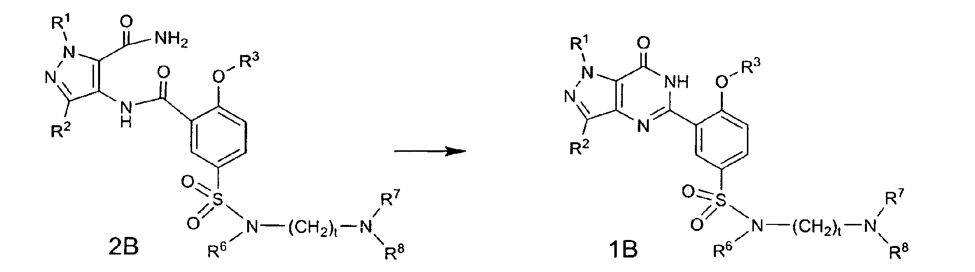 wherein R¹, R², R³, R⁶, R⁷, R⁸ and t are as previously defined in claim 1, and wherein the compounds of Formula 2B are prepared by a process comprising:
transforming the carboxyl group in the compounds of formula 3B into an acyl chloride or mixed anhydride by using thionyl chloride, oxalyl chloride or ethyl chlorformate, and reacting the acyl chloride or mixed anhydride with the compounds of formula 4 to get the compounds of Formula 2B; or
reacting the carboxyl group in the compounds of formula 3B directly with the compounds of formula 4 to get the compounds of Formula 2B.

8. A compound according to any one of the claims 1 to 4 for use in therapy or prophylaxis.

9. Compound for Use as defined in claim 8 which comprises the curative or prophylactic treatment of the diseases of male erectile dysfunction, female sexual dysfunction, premature labour, dysmenorrhoea, benign prostatic hyperplasia (BPH), bladder outlet obstruction, incontinence, stable, unstable and variant (Prinzmetal) angina, hypertension, pulmonary hypertension, congestive heart failure, kidney failure, atherosclerosis, stroke, peripheral vascular disease, conditions of reduced blood vessel patency, inflammatory disease, bronchitis, chronic asthma, allergic asthma, allergic rhinitis, glaucoma or diseases **characterized by** disorders of gut motility.

10. A compound according to any one of the claims 1 to 4 for use in the manufacturing of a medicament for the curative or prophylactic treatment of the diseases of male erectile dysfunction, female sexual dysfunction, premature labour, dysmenorrhoea, benign prostatic hyperplasia (BPH), bladder outlet obstruction, incontinence, stable, unstable and variant (Prinzmetal) angina, hypertension, pulmonary hypertension, congestive heart failure, kidney failure, atherosclerosis, stroke, peripheral vascular disease, conditions of reduced blood vessel patency, inflammatory disease, bronchitis, chronic asthma, allergic asthma, allergic rhinitis, glaucoma or diseases **characterized by** disorders of gut motility.

## Patentansprüche

1. Verbindung mit der Formel (1B) oder ein pharmazeutisch verträgliches
Salz oder Solvat davon, 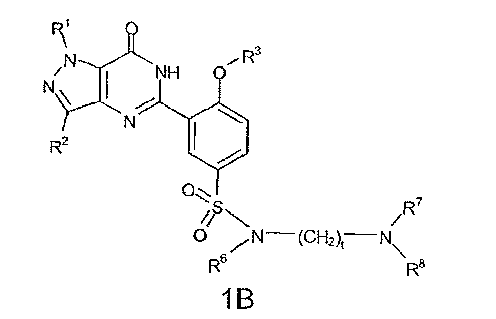 wobei
R¹ H, C₁-C₆-Alkyl, C₃-C₆-Cycloalkyl, C₁-C₃-Alkyl, substituiert durch Halogen, oder C₁-C₃-Alkyl, substituiert durch C₃-C₆-Cycloalkyl, darstellt;
R² C₂-C₆-Alkyl, C₃-C₆-Cycloalkyl, C₁-C₃-Alkyl, substituiert durch Halogen, oder C₁-C₃-Alkyl, substituiert durch C₃-C₆-Cycloalkyl, darstellt;
R³ C₁-C₆-Alkyl, C₃-C₆-Cycloalkyl, C₁-C₃-Alkyl, substituiert durch Halogen, C₁-C₃ Alkyl, substituiert durch C₁-C₃-Alkoxyl, oder C₁-C₃-Alkyl, substituiert durch C₃-C₆-Cycloalkyl, darstellt;
t = 1-5 beträgt;
R⁶ C₁-C₆-Alkyl, C₃-C₆-Cycloalkyl, C₁-C₃-Haloalkyl, C₁-C₃-Alkoxyl, Phenyl, Pyridyl, Furanyl, Pyridazinyl, Pyrazinyl, Imidazolyl, C₁-C₃, substituiert mit Hydroxyl, C₁-C₃-Alkoxyl, Acetoxyl, Phenyl, Pyridyl, Furanyl, Pyridazinyl, Pyrimidinyl, Pyrazinyl, Imidazolyl darstellt; wobei das vorstehend erwähnte Phenyl, Pyridyl, Furanyl, Pyridazinyl, Pyrimidinyl, Pyrazinyl, Imidazolyl mit einem oder mehreren Substituenten substituiert sein kann, die aus Halogen, C₁-C₃-Alkyl, C₁-C₃-Alkoxyl ausgewählt werden; und
R⁷ und R⁸ unabhängig voneinander H, C₁-C₆-Alkyl, C₃-C₆-Cycloalkyl, C₁-C₃-Haloalkyl, C₁-C₆-Alkoxyl, C₁-C₃-Alkyl, substituiert mit Hydroxyl, Acetoxyl, C₁-C₃-Alkoxyl darstellen, oder R⁷ und R⁸ bilden zusammen mit dem Stickstoff, an den sie gebunden sind, einen vier- bis achtgliedrigen heterozyklischen Ring, wie beispielsweise Morpholin, Piperidin, Pyrrol, Piperazin; wobei der vorstehend erwähnte heterozyklische Ring mit einem oder mehreren Substituenten substituiert sein kann, die aus Halogen, C₁-C₃-Alkyl, C₃-C₆-Cycloalkyl, C₁-C₃-Haloalkyl und C₁-C₃-Alkoxyl ausgewählt werden.

2. Verbindung nach Anspruch 1, wobei:
R¹ C₁-C₄-Alkyl oder C₃-C₆-Cycloalkyl darstellt;
R² C₂-C₄-Alkyl oder C₃-C₆-Cycloalkyl darstellt;
R³ C₁-C₃-Alkyl oder C₁-C₃-Alkyl, substituiert mit C₁-C₃-Alkoxyl, darstellt;
t = 2-3 beträgt;
R⁶ C₁-C₃-Alkyl, Phenyl, Pyridyl oder C₁-C₃, substituiert mit Hydroxyl, C₁-C₃-Alkoxyl, Acetoxyl, Phenyl, Pyridyl, darstellt; und
R⁷ und R⁸ zusammen mit dem Stickstoff, an den sie gebunden sind, einen heterozyklischen Morpholin-, Piperidin- oder Pyrrolring bilden.

3. Verbindung nach Anspruch 2, wobei
R¹ Methyl oder Ethyl darstellt;
R² Ethyl und n-Propyl darstellt;
R³ Ethyl, n-Propyl oder Methyloxyethyl darstellt;
t = 2-3 beträgt,
R⁶ Methyl, Ethyl, Benzyl, Pyridylmethyl oder C₁-C₃, substituiert mit Hydroxyl, C₁-C₃-Alkoxyl, Acetoxyl, darstellt; und
R⁷ und R⁸ zusammen mit dem Stickstoff, an den sie gebunden sind, einen heterozyklischen Morpholin-, Piperidin- oder Pyrrolring bilden.

4. Verbindung nach Anspruch 1, die aus der folgenden Gruppe ausgewählt wird:
1-Methyl-5-{2-ethoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-on (Verbindung von Beispiel 13);
1-Methyl-5-{2-propoxy-5-[1-(2-diethylaminoethyl)-1-(2-hydroxyethyl)amidosulfonyl]phenyl}-3-n-propyl-1,6-dihydro-7H-pyrazolo[4,3-d]pyrimidin-7-on (Verbindung von Beispiel 14);
1-Methyl-5-{2-ethoxy-5-[1-(2-diethylaminoethyl)-1-(2-hydroxyethoxyethyl)amidosulfonyl]phenyl}-3-n-propyl-1,6-dihydro-7H-pyrazolo[4,3-d]pyrimidin-7-on (Verbindung von Beispiel 16);
1-Ethyl-5-{2-ethoxy-5-{[1-ethyl-1-[2-(1-ethyl-1-(2-acetoxyethyl))amino]ethyl]amidosulfonyl{phenyl}-3-n-propyl-1,6-dihydro-7H-pyrazolo[4,3-d]pyrimidin-7-on (Verbindung von Beispiel 19);
1-Methyl-5-{2-ethoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-on (Verbindung von Beispiel 21);
1-Methyl-5-{2-propoxy-5-{[1-methyl-1-[2-(1-methyl-1-(2-hydroxyethyl))amino]ethyl]amidosulfonyl}phenyl}-3-n-propyl-1,6-dihydro-7H-pyrazolo[4,3-d]pyrimidin-7-on (Verbindung von Beispiel 23);
1-Methyl-5-{2-propoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 24);
1-Methyl-5-{2-ethoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 25);
1-Methyl-5-{2-methoxy-5-[[1-benzyl-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 26);
1-Methyl-5-{2-ethoxy-5-[[1-benzyl-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 27);
1-Methyl-5-{2-ethoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 28);
1-Methyl-5-{2-ethoxy-5-[[1-(2-hydroxyethyl)-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 29);
1-Methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 35);
1-Methyl-5-{2-ethoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 36);
1-Methyl-5-{2-propoxy-5-[[1-ethyl-1-(2-morpholin-1-yl)ethyl]amidosulfonyl]phenyl-3-propyl-1,6-dihydro-7H-pyrazolo[4,3-d]pyrimidin-7-on (Verbindung von Beispiel 41);
1-Methyl-5-{2-ethoxy-5-[[1-ethyl-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 43);
1-Methyl-5-{2-propoxy-5-[[1-methyl-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 53);
1-Methyl-5-{2-propoxy-5-[[1-ethyl-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 54);
1-Methyl-5-{2-propoxy-5-[[1-(4-fluorobenzyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 56);
1-Methyl-5-{2-propoxy-5-[[1-(2-pyridylmethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 60);
1-Methyl-5-{2-ethoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 61);
1-Methyl-5-{2-propoxy-5-[[1-(3-pyridylmethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 62);
1-Methyl-5-{2-propoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 63);
1-Methyl-5-{2-ethoxy-5-[[1-(4-pyridylmethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 64);
1-Methyl-5-{2-propoxy-5-[[1-(2-furanylmethyl)-1-(2-morpholin-1-yl)ethyl]amidosulfonyl]phenyl}-3-ethyl-1,6-dihydro-7H-pyrazolo[4,3-d]pyrimidin-7-on (Verbindung von Beispiel 72);
1-Methyl-5-{2-propoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 78);
1-Methyl-5-{2-ethoxy-5-[[1-methyl-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 79);
1-Methyl-5-{2-propoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 80);
1-Methyl-5-{2-ethoxy-5-[[1-methyl-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 81);
1-Methyl-5-{2-ethoxy-5-[[1-benzyl-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 82);
1-Methyl-5-{2-propoxy-5-[[1-benzyl-1-(2-piperidin-I-yl)ethyl]amidosulfonyl]phenyl}-3-n-propyl-1,6-dihydro-7H-pyrazolo[4,3-d]pyrimidin-7-on (Verbindung von Beispiel 83);
1-Methyl-5-{2-propoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 84);
1-Methyl-5-{2-ethoxy-5-[[1-benzyl-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 85);
1-Methyl-5-{2-propoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 87);
1-Methyl-5-{2-ethoxy-5-[[1-(2-pyridylmethyl)-1-(2-piperidin-1-yl)ethyl]amidosulfonyl]phenyl}-3-n-propyl-1,6-dihydro-7H-pyrazolo[4,3-d]pyrimidin-7-on (Verbindung von Beispiel 88);
1-Methyl-5-{2-propoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 89);
1-Methyl-5-{2-ethoxy-5-[[1-(3-pyridylmethyl)-1-(2-pyrrolidin-1-yl)ethyl]amidosulfonyl]phenyl}-3-n-propyl-1,6-dihydro-7H-pyrazolo[4,3-d]pyrimidin-7-on (Verbindung von Beispiel 90);
1-Methyl-5-{2-propoxy-5-[[1-(2-hydroxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 95);
1-Methyl-5-{2-propoxy-5-[[1-(2-acetoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 96);
1-Methyl-5-{2-propoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 98);
1-Methyl-5-{2-ethoxy-5-[[1-(2-methoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 100);
1-Methyl-5-{2-propoxy-5-[[1-(2-hydroxyethoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 104);
1-Methyl-5-{2-propoxy-5-[[1-(2-methoxyethoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 105);
1-Methyl-5-{2-propoxy-5-[[1-methyl-1-(3-dimethylaminopropyl)]amidosulfonyl]phenyl}-3-n-propyl-1,6-dihydro-7H-pyrazolo[4,3-d]pyrimidin-7-on (Verbindung von Beispiel 110);
1-Methyl-5-{2-propoxy-5-[[1-(2-ethoxyethyl)-1-(2-morpholin-1-yl)ethyl]amidosulfonyl]phenyl}-3-n-propyl-1,6-dihydro-7H-pyrazolo[4,3-d]pyrimidin-7-on (Verbindung von Beispiel 111);
1-Methyl-5-{2-propoxy-5-[[1-ethyl-1-(3-morpholin-1-yl)propyl]amidosulfonyl]phenyl}-3-n-propyl-1,6-dihydro-7H-pyrazolo[4,3-d]pyrimidin-7-on (Verbindung von Beispiel 112);
1-Methyl-5-{2-propoxy-5-[[1-benzyl-1-(3-morpholin-1-yl)propyl]amidosulfonyl]phenyl}-3-n-propyl-1,6-dihydro-7H-pyrazolo[4,3-d]pyrimidin-7-on (Verbindung von Beispiel 113);
1-Methyl-5-{2-propoxy-5-[[1-benzyl-1-(4-morpholin-1-yl)butyl]amidosulfonyl]phenyl}-3-n-propyl-1,6-dihydro-7H-pyrazolo[4,3-d]pyrimidin-7-on (Verbindung von Beispiel 117).

5. Pharmazeutische Zusammensetzung mit einer Verbindung der Formel 1 B nach einem der Ansprüche 1 bis 4 oder einem pharmazeutisch verträglichen Salz oder Solvat davon, zusammen mit einem oder mehreren pharmazeutisch verträglichen Trägerstoffen.

6. Zusammensetzung nach Anspruch 5, wobei die effektive Dosis 1-500 mg/Tag, vorzugsweise 10-100 mg/Tag beträgt.

7. Verfahren zum Herstellen der Verbindungen der Fromel 1B nach Anspruch 1 oder einem Prodrug davon oder einem pharmazeutisch verträglichen Salz oder Solvat davon, wobei die Verbindungen der Formel 1 B gemäß dem folgenden Schema hergestellt werden: 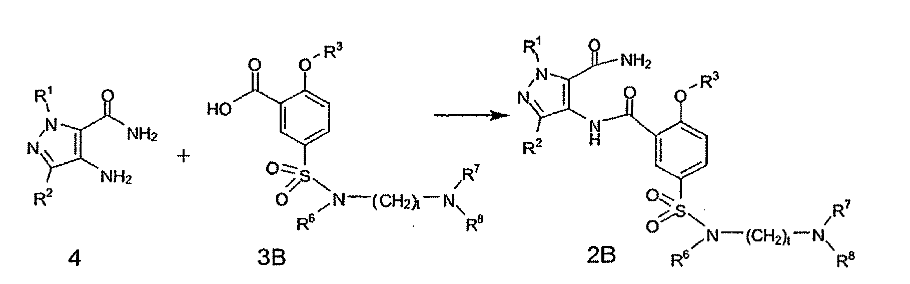 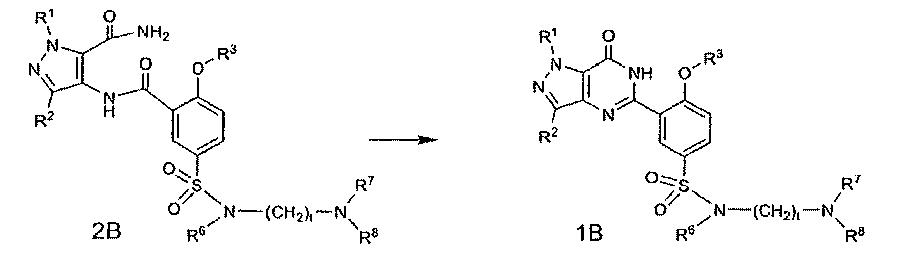 wobei R¹, R², R³, R⁶, R⁷, R⁸ und t wie vorstehend in Anspruch 1 definiert sind, und wobei die Verbindungen der Formel 2B durch einen Prozess hergestellt werden, der die Schritte aufweist:
Umwandeln der Carboxylgruppe in den Verbindungen der Formel 3B in ein Acylchlorid oder ein Misch-Anhydrid unter Verwendung von Thionylchlorid, Oxalylchlorid oder Ethylchlorformiat, und Veranlassen einer Reaktion zwischen dem Acylchlorid oder dem Misch-Anhydrid mit den Verbindungen von Formel 4, um die Verbindungen der Formel 2B zu erhalten;
oder
Veranlassen einer direkten Reaktion zwischen der Carboxylgruppe in den Verbindungen von Formel 3B mit den Verbindungen von Formel 4, um die Verbindungen der Formel 2B zu erhalten..

8. Verbindung nach einem der Ansprüche 1 bis 4 zur Anwendung in einer Therapie oder Prophylaxe.

9. Verbindung zur Anwendung nach Anspruch 8, wobei die Anwendung die medizinische oder prophylaktische Behandlung von Störungen einer männlichen erektilen Dysfunktion, einer weiblichen sexuellen Dysfunktion, von Frühwehen, Dysmenorrhö, gutartiger Prostatahyperplasie (BPH), Blasenausgangobstruktion, Inkontinenz, stabiler, instabiler und Variant(Prinzmetal-)angina, Bluthochdruck, Lungenhochdruck, Herzinsuffizienz, Nierenversagen, Arteriosklerose, Schlaganfall, peripherer Gefäßerkrankung, Zuständen verminderter Blutgefäßdurchgängigkeit, Entzündungskrankheiten, Bronchitis, chronischem Asthma, allergischem Asthma, allergischer Nasenschleimhautentzündung, grünem Star oder Krankheiten aufweist, die durch Störungen der Darmbeweglichkeit **gekennzeichnet** sind.

10. Verbindung nach einem der Ansprüche 1 bis 4 zur Verwendung bei der Herstellung eines Medikaments zur medizinischen oder prophylaktischen Behandlung von Störungen einer männlichen erektilen Dysfunktion, einer weiblichen sexuellen Dysfunktion, von Frühwehen, Dysmenorrhö, gutartiger Prostatahyperplasie (BPH), Blasenausgangobstruktion, Inkontinenz, stabiler, instabiler und Variant(Prinzmetal-)angina, Bluthochdruck, Lungenhochdruck, Herzinsuffizienz, Nierenversagen, Arteriosklerose, Schlaganfall, peripherer Gefäßerkrankung, Zuständen verminderter Blutgefäßdurchgängigkeit, Entzündungskrankheiten, Bronchitis, chronischem Asthma, allergischem Asthma, allergischer Nasenschleimhautentzündung, grünem Star oder Krankheiten, die durch Störungen der Darmbeweglichkeit **gekennzeichnet** sind.

## Revendications

1. Composé de formule (1B), ou un de ses sels ou solvates pharmaceutiquement acceptables: 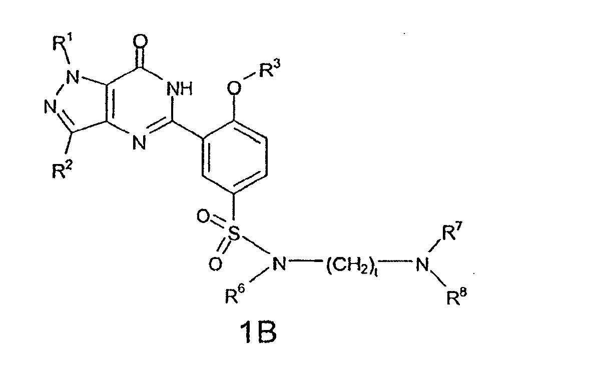 où
R¹ représente H, un alkyle en C₁-C₆, cycloalkyle en C₃-C₆, alkyle en C₁-C₃ substitué par halogène, ou alkyle en C₁-C₃ substitué par cycloalkyle en C₃-C₆;
R² représente un alkyle en C₂-C₆, cycloalkyle en C₃-C₆, alkyle en C₁-C₃ substitué par halogène, ou alkyle en C₁-C₃ substitué par cycloalkyle en C₃-C₆;
R³ représente un alkyle en C₁-C₆, cycloalkyle en C₃-C₆, alkyle en C₁-C₃ substitué par halogène, alkyle en C₁-C₃ substitué par alcoxy en C₁-C₃ ou alkyle en C₁-C₃ substitué par cycloalkyle en C₃-C₆;
t = 1-5;
R⁶ représente un alkyle en C₁-C₆, cycloalkyle en C₃-C₆, halogénoalkyle en C₁-C₃, alcoxy en C₁-C₃, phényle, pyridyle, furanyle, pyridazinyle, pyrazinyle, imidazolyle, C₁-C₃ substitué par hydroxy, alcoxy en C₁-C₃, acétoxy, phényle, pyridyle, furanyle, pyridazinyle, pyrimidinyle, pyrazinyle, imidazolyle; les groupes phényle, pyridyle, furanyle, pyridazinyle, pyrimidinyle, pyrazinyle, imidazolyle ci-dessus éventuellement substitués par un ou plusieurs substituants choisis parmi halogène, alkyle en C₁-C₃, alcoxy en C₁-C₃;
R⁷ et R⁸ représentent indépendamment H, un alkyle en C₁-C₆, cycloalkyle en C₃-C₆, halogénoalkyle en C₁-C₃, alcoxy en C₁-C₆, alkyle en C₁-C₃ substitué par hydroxy, acétoxy, alcoxy en C₁-C₃, ou R⁷ et R⁸ ensemble, avec l'azote auquel ils sont liés, forment un cycle hétérocyclique de 4 à 8 chaînons, comprenant la morpholine, la pipéridine, le pyrrole, la pipérazine; le cycle hétérocyclique ci- dessus éventuellement substitué par un ou plusieurs substituants choisis halogène, alkyle en C₁-C₃, cycloalkyle en C₃-C₆, halogénoalkyle en C₁-C₃, alcoxy en C₁-C_{3.}

2. Composé selon la revendication 1, dans lequel:
R¹ représente un alkyle en C₁-C₄ ou cycloalkyle en C₃-C₆;
R² représente un alkyle en C₂-C₄ ou cycloalkyle en C₃-C₆;
R³ représente un alkyle en C₁-C₃, alkyle en C₁-C₃ substitué par alcoxy en C₁-C₃;
t = 2-3;
R⁶ représente un alkyle en C₁-C₃, phényle, pyridyle ou C₁-C₃ substitué par hydroxy, alcoxy en C₁-C₃, acétoxy, phényle, pyridyle;
R⁷ et R⁸ ensemble, avec l'azote auquel ils sont liés, forment un cycle hétérocyclique morpholine, pipéridine ou pyrrole.

3. Composé selon la revendication 2, dans lequel:
R¹ représente un méthyle ou éthyle;
R² représente un éthyle et n-propyle;
R³ représente un éthyle, n-propyle ou méthyloxyéthyle;
t = 2-3;
R⁶ représente un méthyle, éthyle, benzyle, pyridylméthyle, ou C₁-C₃ substitué par hydroxy, alcoxy en C₁-C₃, acétoxy;
R⁷ et R⁸ ensemble, avec l'azote auquel ils sont liés, forment un cycle hétérocyclique morpholine, pipéridine ou pyrrole.

4. Composé selon la revendication 1, qui est choisi dans le groupe constitué par:
la 1-méthyl-5-{2-éthoxy-5-[1-(2-diéthylaminoéthyl)-1-(2-hydroxyéthyl)amido-sulfonyl]phényl}-3-n-propyl-1,6-dihydro-7H-pyrazolo[4,3-d]pyrimidin-7-one (composé de l'exemple 13)
la 1-méthyl-5-{2-propoxy-5-[1-(2-diéthylaminoéthyl)-1-(2-hydroxyéthyl)amido-sulfonyl]phényl}-3-n-propyl-1,6-dihydro-7H-pyrazolo[4,3-d]pyrimidin-7-one (composé de l'exemple 14)
la 1-méthyl-5-{2-éthoxy-5-[1-(2-diéthylaminoéthyl)-1-(2-hydroxyéthoxyéthyl)-amidosulfonyl]phényl}-3-n-propyl-1,6-dihydro-7H-pyrazolo[4,3-d]pyrimidin-7-one (composé de l'exemple 16)
la 1-éthyl-5-{2-éthoxy-5-{[1-éthyl-1-[2-(1-éthyl-1-(2-acétoxyéthyl))amino]éthyl]-amidosulfonyl}phényl}-3-n-propyl-1,6-dihydro-7H-pyrazolo[4,3-d]pyrimidin-7-one (composé de l'exemple 19)
la 1-méthyl-5-{2-éthoxy-5-{[1-méthyl-1-[2-(1-méthyl-1-(2-hydroxyéthyl))amino]-éthyl]amidosulfonyl}phényl}1-3-n-propyl-1,6-dihydro-7H-pyrazolo[4,3-d]pyrimidin-7-one (composé de l'exemple 21)
la 1-méthyl-5-{2-propoxy-5-{[1-méthyl-1-[2-(1-méthyl-1-(2-hydroxyéthyl))amino]-éthyl]amidosulfonyl}phényl}-3-n-propyl-1,6-dihydro-7H-pyrazolo[4,3-d]pyrimidin-7-one (composé de l'exemple 23)
la 1-méthyl-5-{2-propoxy-5-[[1-benzyl-1-(2-morpholin-1-yl)éthyl]amidosulfonyl]-phényl}-3-n-propyl-1,6-dihydro-7H-pyrazolo[4,3-d]pyrimidin-7-one (composé de l'exemple 24)
la 1-méthyl-5-{2-éthoxy-5-[[1-benzyl-1-(2-morpholin-1-yl)éthyl]amidosulfonyl]-phényl}-3-n-propyl-1,6-dihydro-7H-pyrazolo[4,3-d]pyrimidin-7-one (composé de l'exemple 25)
la 1-méthyl-5-{2-méthoxy-5-[[1-benzyl-1-(2-morpholin-1-yl)éthyl]amidosulfonyl]-phényl}-3-n-propyl-1,6-dihydro-7H-pyrazolo[4,3-d]pyrimidin-7-one (composé de l'exemple 26)
la 1-méthyl-5-{2-éthoxy-5-[[1-benzyl-1-(2-morpholin-1-yl)éthyl]amidosulfonyl]-phényl}-3-éthyl-1,6-dihydro-7H-pyrazolo[4,3-d]pyrimidin-7-one (composé de l'exemple 27)
la 1-méthyl-5-{2-éthoxy-5-[[1-(2-hydroxyéthoxyéthyl)-1-(2-morpholin-1-yl)éthyl]-amidosulfonyl]phényl}-3-n-propyl-1,6-dihydro-7H-pyrazolo[4,3-d]pyrimidin-7-one (composé de l'exemple 28)
la 1-méthyl-5-{2-éthoxy-5-[[1-(2-hydroxyéthyl)-1-(2-pipéridin-1-yl)éthyl]amido-sulfonyl]phényl}-3-n-propyl-1,6-dihydro-7H-pyrazolo[4,3-d]pyrimidin-7-one (composé de l'exemple 29)
la 1-méthyl-5-{2-propoxy-5-[[1-méthyl-1-(2-morpholin-1-yl)éthyl]amidosulfonyl]-phényl}-3-éthyl-1,6-dihydro-7H-pyrazolo[4,3-d]pyrimidin-7-one (composé de l'exemple 35)
la 1-méthyl-5-{2-éthoxy-5-[[1-méthyl-1-(2-morpholin-1-yl)éthyl]amidosulfonyl]-phényl}-3-éthyl-1,6-dihydro-7H-pyrazolo[4,3-d]pyrimidin-7-one (composé de l'exemple 36)
la 1-méthyl-5-{2-propoxy-5-[[1-éthyl-1-(2-morpholin-1-yl)éthyl]amidosulfonyl]-phényl}-3-propyl-1,6-dihydro-7H-pyrazolo[4,3-d]pyrimidin-7-one (composé de l'exemple 41)
la 1-méthyl-5-{2-éthoxy-5-[[1-éthyl-1-(2-morpholin-1-yl)éthyl]amidosulfonyl]-phényl}-3-n-propyl-1,6-dihydro-7H-pyrazolo[4,3-d]pyrimidin-7-one (composé de l'exemple 43)
la 1-méthyl-5-{2-propoxy-5-[[1-méthyl-1-(2-morpholin-1-yl)éthyl]amidosulfonyl]-phényl}-3-éthyl-1,6-dihydro-7H-pyrazolo[4,3-d]pyrimidin-7-one (composé de l'exemple 53)
la 1-méthyl-5-{2-propoxy-5-[[1-éthyl-1-(2-morpholin-1-yl)éthyl]amidosulfonyl]-phényl}-3-éthyl-1,6-dihydro-7H-pyrazolo[4,3-d]pyrimidin-7-one (composé de l'exemple 54)
la 1-méthyl-5-{2-propoxy-5-[[1-(4-fluorobenzyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 56)
la 1-méthyl-5-{2-propoxy-5-[[1-(2-pyridylméthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 60)
la 1-méthyl-5-{2-éthoxy-5-[[1-(3-pyridylméthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 61)
la 1-méthyl-5-{2-propoxy-5-[[1-(3-pyridylméthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 62)
la 1-méthyl-5-{2-propoxy-5-[[1-(4-pyridylméthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 63)
la 1-méthyl-5-{2-éthoxy-5-[[1-(4-pyridylméthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 64)
la 1-méthyl-5-{2-propoxy-5-[[1-(2-furanylméthyl)-1-(2-morpholin-1-yl)éthyl]amido-sulfonyl]phényl}-3-éthyl-1,6-dihydro-7H-pyrazolo[4,3-d]pyrimidin-7-one (composé de l'exemple 72)
la 1-méthyl-5-{2-propoxy-5-[[1-méthyl-1-(2-pipéridin-1-yl)éthyl]amidosulfonyl]-phényl}-3-n-propyl-1,6-dihydro-7H-pyrazolo[4,3-d]pyrimidin-7-one (composé de l'exemple 78)
la 1-méthyl-5-{2-éthoxy-5-[[1-méthyl-1-(2-pipéridin-1-yl)éthyl]amidosulfonyl]-phényl}-3-n-propyl-1,6-dihydro-7H-pyrazolo[4,3-d]pyrimidin-7-one (composé de l'exemple 79)
la 1-méthyl-5-{2-propoxy-5-[[1-méthyl-1-(2-pyrrolidin-1-yl)éthyl]amidosulfonyl]-phényl}-3-n-propyl-1,6-dihydro-7H-pyrazolo[4,3-d]pyrimidin-7-one (composé de l'exemple 80)
la 1-méthyl-5-{2-éthoxy-5-[[1-méthyl-1-(2-pyrrolidin-1-yl)éthyl]amidosulfonyl]-phényl}-3-n-propyl-1,6-dihydro-7H-pyrazolo[4,3-d]pyrimidin-7-one (composé de l'exemple 81)
la 1-méthyl-5-{2-éthoxy-5-[[1-benzyl-1-(2-pipéridin-1-yl)éthyl]amidosulfonyl]-phényl}-3-n-propyl-1,6-dihydro-7H-pyrazolo[4,3-d]pyrimidin-7-one (composé de l'exemple 82)
la 1-méthyl-5-{2-propoxy-5-[[1-benzyl-1-(2-pipéridin-1-yl)éthyl]amidosulfonyl]-phényl}-3-n-propyl-1,6-dihydro-7H-pyrazolo[4,3-d]pyrimidin-7-one (composé de l'exemple 83)
la 1-méthyl-5-{2-propoxy-5-[[1-benzyl-1-(2-pyrrolidin-1-yl)éthyl]amidosulfonyl]-phényl}-3-n-propyl-1,6-dihydro-7H-pyrazolo[4,3-d]pyrimidin-7-one (composé de l'exemple 84)
la 1-méthyl-5-{2-éthoxy-5-[[1-benzyl-1-(2-pyrrolidin-1-yl)éthyl]amidosulfonyl]-phényl}-3-n-propyl-1,6-dihydro-7H-pyrazolo[4,3-d]pyrimidin-7-one (composé de l'exemple 85)
la 1-méthyl-5-{2-propoxy-5-[[1-(2-pyridylméthyl)-1-(2-pipéridin-1-yl)éthyl]amido-sulfonyl]phényl}-3-n-propyl-1,6-dihydro-7H-pyrazolo[4,3-d]pyrimidin-7-one (composé de l'exemple 87)
la 1-méthyl-5-{2-éthoxy-5-[[1-(2-pyridylméthyl)-1-(2-pipéridin-1-yl)éthyl]amido-sulfonyl]phényl}-3-n-propyl-1,6-dihydro-7H-pyrazolo[4,3-d]pyrimidin-7-one (composé de l'exemple 88)
la 1-méthyl-5-{2-propoxy-5-[[1-(3-pyridylméthyl)-1-(2-pyrrolidin-1-yl)éthyl]amido-sulfonyl]phényl}-3-n-propyl-1,6-dihydro-7H-pyrazolo[4,3-d]pyrimidin-7-one (composé de l'exemple 89)
la 1-méthyl-5-{2-éthoxy-5-[[1-(3-pyridylméthyl)-1-(2-pyrrolidin-1-yl)éthyl]amido-sulfonyl]phényl}-3-n-propyl-1,6-dihydro-7H-pyrazolo[4,3-d]pyrimidin-7-one (composé de l'exemple 90)
la 1-méthyl-5-{2-propoxy-5-[[1-(2-hydroxyéthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 95)
la 1-méthyl-5-{2-propoxy-5-[[1-(2-acétoxyéthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 96)
la 1-méthyl-5-{2-propoxy-5-[[1-(2-méthoxyéthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 98)
la 1-méthyl-5-{2-éthoxy-5-[[1-(2-méthoxyéthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 100)
la 1-méthyl-5-{2-propoxy-5-[[1-(2-hydroxyéthoxyéthyl)-1-(2-morpholin-1-yl)éthyl]-amidosulfonyl]phényl}-3-n-propyl-1,6-dihydro-7H-pyrazolo[4,3-d]pyrimidin-7-one (composé de l'exemple 104)
la 1-méthyl-5-{2-propoxy-5-[[1-(2-méthoxyéthoxyéthyl)-1-(2-morpholin-1-yl)éthyl]-amidosulfonyl]phényl}-3-n-propyl-1,6-dihydro-7H-pyrazolo[4,3-d]pyrimidin-7-one (composé de l'exemple 105)
la 1-méthyl-5-{2-propoxy-5-[[1-méthyl-1-(3-diméthylaminopropyl)]amidosulfonyl]-phényl}-3-n-propyl-1,6-dihydro-7H-pyrazolo[4,3-d]pyrimidin-7-one (composé de l'exemple 110)
la 1-méthyl-5-{2-propoxy-5-[[1-(2-éthoxyéthyl)-1-(2-morpholin-1-yl)éthyl]amido-sulfonyl]phényl}-3-n-propyl-1,6-dihydro-7H-pyrazolo[4,3-d]pyrimidin-7-one (composé de l'exemple 111)
la 1-méthyl-5-{2-propoxy-5-[[1-éthyl-1-(3-morpholin-1-yl)propyl]amidosulfonyl]-phényl}-3-n-propyl-1,6-dihydro-7H-pyrazolo[4,3-d]pyrimidin-7-one (composé de l'exemple 112)
la 1-méthyl-5-{2-propoxy-5-[[1-benzyl-1-(3-morpholin-1-yl)propyl]amidosulfonyl]-phényl}-3-n-propyl-1,6-dihydro-7H-pyrazolo[4,3-d]pyrimidin-7-one (composé de l'exemple 113)
la 1-méthyl-5-{2-propoxy-5-[[1-benzyl-1-(4-morpholin-1-yl)butyl]amidosulfonyl]-phényl}-3-n-propyl-1,6-dihydro-7H-pyrazolo[4,3-d]pyrimidin-7-one (composé de l'exemple 117).

5. Composition pharmaceutique comprenant un composé de formule 1B tel que défini dans l'une quelconque des revendications 1 à 4, ou un de ses sels ou solvates pharmaceutiquement acceptables, avec un ou plusieurs excipients pharmaceutiquement acceptables.

6. Composition selon la revendication 5, dont la posologie efficace est de 1-500 mg/jour, de préférence 10-100 mg/jour.

7. Procédé de préparation des composés de formule 1B selon la revendication 1, ou d'un de ses promédicaments, ou d'un de ses sels ou solvates pharmaceutiquement acceptables, où les composés de formule 1B sont préparés selon le schéma suivant: 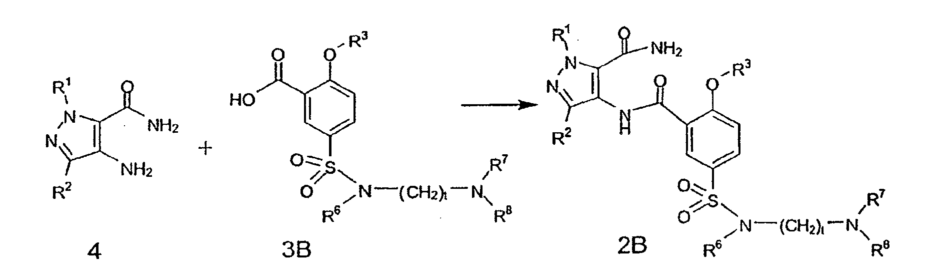 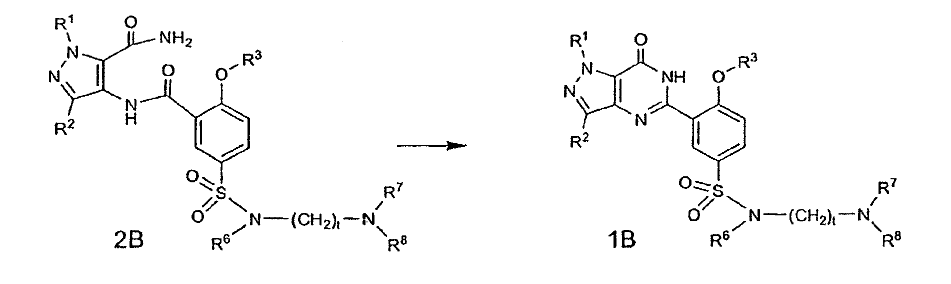 où R¹, R², R³, R⁶, R⁷, R⁸ et t sont tels que définis précédemment dans la revendication 1, et où les composés de formule 2B sont préparés par un procédé comprenant:
la transformation du groupe carboxyle dans les composés de formule 3B en un chlorure d'acyle ou un anhydride mixte à l'aide de chlorure de thionyle, de chlorure d'oxalyle ou de chloroformate d'éthyle, et la réaction du chlorure d'acyle ou de l'anhydride mixte avec les composés de formule 4 pour donner les composés de formule 2B; ou
la réaction du groupe carboxyle dans les composés de formule 3B directement avec les composés de formule 4 pour donner les composés de formule 2B.

8. Composé selon l'une quelconque des revendications 1 à 4, pour son utilisation en thérapie ou en prophylaxie.

9. Composé pour son utilisation telle que définie dans la revendication 8, qui comprend le traitement curatif ou prophylactique des maladies du dysfonctionnement érectile masculin, du dysfonctionnement sexuel féminin, du travail prématuré, de la dysménorrhée, de l'hyperplasie bénigne de la prostate (HBP), de l'obstruction du col de la vessie, de l'incontinence, de l'angor stable, instable et variant (Prinzmetal), de l'hypertension, de l'hypertension pulmonaire, de l'insuffisance cardiaque congestive, de l'insuffisance rénale, de l'athérosclérose, de l'AVC, des maladies vasculaires périphériques, des états de perméabilité réduite des vaisseaux sanguins, des maladies inflammatoires, de la bronchite, de l'asthme chronique, de l'asthme allergique, de la rhinite allergique, du glaucome ou des maladies **caractérisées par** des troubles de la motilité intestinale.

10. Composé selon l'une quelconque des revendications 1 à 4, pour son utilisation dans la fabrication d'un médicament destiné au traitement curatif ou prophylactique des maladies du dysfonctionnement érectile masculin, du dysfonctionnement sexuel féminin, du travail prématuré, de la dysménorrhée, de l'hyperplasie bénigne de la prostate (HBP), de l'obstruction du col de la vessie, de l'incontinence, de l'angor stable, instable et variant (Prinzmetal), de l'hypertension, de l'hypertension pulmonaire, de l'insuffisance cardiaque congestive, de l'insuffisance rénale, de l'athérosclérose, de l'AVC, des maladies vasculaires périphériques, des états de perméabilité réduite des vaisseaux sanguins, des maladies inflammatoires, de la bronchite, de l'asthme chronique, de l'asthme allergique, de la rhinite allergique, du glaucome ou des maladies **caractérisées par** des troubles de la motilité intestinale.
